# EUROPEAN PATENT SPECIFICATION

(11) **EP 1 911 459 B1**
(45) Date of publication and mention of the grant of the patent: **02.12.2015**
(21) Application number: 06781920.1
(22) Date of filing: 28.07.2006
(51) Int. Cl.: A61K 38/22, A61K 48/00, A61P 7/10, C07K 14/47, C12N 15/09, C12P 21/02, C12R 1/91

(54) **LYMPHANGIOGENESIS PROMOTER**
MITTEL ZUR FÖRDERUNG DER LYMPHANGIOGENESE
PROMOTEUR DE LYMPHANGIOGENÈSE

(30) Priority: 28.07.2005 JP 2005219410; 29.05.2006 JP 2006148970
(43) Date of publication of application: 16.04.2008
(73) Proprietor: AnGes MG, Inc., Ibaraki-shi, Osaka 5670085 (JP)
(72) Inventor: MORISHITA, Ryuichi, 5650851 (JP); KANEDA, Yasufumi, 5620031 (JP); NAKAGAMI, Hironori, 5670048 (JP); SAITO, Yukihiro, Asahikawa-shi, Hokkaido 0788313 (JP)
(74) Representative: Denison, Christopher Marcus
(86) International application number: PCT/JP2006/315010
(87) International publication number: WO 2007/013603

(56) References cited:
- WO-A-2006/105511
- KAJIYA K ET AL: "Hepatocyte growth factor promotes lymphatic vessel formation and function" JOURNAL OF INVESTIGATIVE DERMATOLOGY, vol. 124, no. 4, Suppl. S, April 2005 (2005-04), page A1, XP002538898 & 66TH ANNUAL MEETING OF THE SOCIETY-FOR-INVESTIGATIVE-DERMATOLOGY; ST LOUIS, MO, USA; MAY 04 -07, 2005 ISSN: 0022-202X
- HIRAOKA KAZUYA ET AL: "Enhanced therapeutic angiogenesis by cotransfection of prostacyclin synthase gene or optimization of intramuscular injection of naked plasmid DNA." CIRCULATION, vol. 108, no. 21, 25 November 2003 (2003-11-25), pages 2689-2696, XP002538899 ISSN: 0009-7322
- KAJIYA KENTARO ET AL: "Hepatocyte growth factor promotes lymphatic vessel formation and function" EMBO JOURNAL, OXFORD UNIVERSITY PRESS, SURREY, GB, vol. 24, no. 16, 28 July 2005 (2005-07-28), pages 2885-2895, XP002390312 ISSN: 0261-4189
- JIANG W G ET AL: "THE POTENTIAL LYMPHANGIOGENIC EFFECTS OF HEPATOCYTE GROWTH FACTOR/SCATTER FACTOR IN VITRO AND IN VIVO" INTERNATIONAL JOURNAL OF MOLECULAR MEDICINE, SPANDIDOS, ATHENS, GR, vol. 16, no. 4, 1 October 2005 (2005-10-01), pages 723-728, XP008066625 ISSN: 1107-3756
- ALITALO K ET AL: "Molecular mechanisms of lymphangiogenesis in health and disease" CANCER CELL, CELL PRESS, US, vol. 1, no. 3, 1 April 2002 (2002-04-01), pages 219-227, XP002272726 ISSN: 1535-6108
- DATABASE Geneseq [Online] 30 December 2004 (2004-12-30), "Human hepatocyte growth factor (HGF) polypeptide encoding cDNA." XP002538901 retrieved from EBI accession no. GSN:ADT08218 Database accession no. ADT08218
- DATABASE JPO Proteins [Online] 27 July 1999 (1999-07-27), "Hepatocyte growth factor." XP002538902 retrieved from EBI accession no. JPOP:E63746 Database accession no. E63746
- DATABASE Geneseq [Online] 4 December 2003 (2003-12-04), "Hepatocyte growth factor (HGF) receptor related DNA, SEQ ID No 1." XP002538903 retrieved from EBI accession no. GSN:ADB61585 Database accession no. ADB61585
- DATABASE UniProt [Online] 1 August 1988 (1988-08-01), "RecName: Full=Hepatocyte growth factor receptor; Short=HGF receptor; EC=<A HREF="http://srs.ebi.ac.uk/srsbin/cgi-bin/ wgetz?[enzyme-ECNumber:2.7.10.1]+-e">2.7.1 0.1</A>; AltName: Full=Scatter factor receptor; Short=SF receptor; AltName: Full=HGF/SF receptor; AltName: Full=Met proto-oncogene tyrosine k" XP002538904 retrieved from EBI accession no. UNIPROT:P08581 Database accession no. P08581
- SAITO YUKIHIRO ET AL: "Transfection of human hepatocyte growth factor gene ameliorates secondary lymphedema via promotion of lymphangiogenesis" CIRCULATION, vol. 114, no. 11, September 2006 (2006-09), pages 1177-1184, XP002538900 ISSN: 0009-7322
- KAJIYA K. ET AL.: 'Hepatocypte growth factor promotes lymphatic vessel formation and function' THE JOURNAL OF INVESTIGATIVE DERMATOLOGY vol. 124, no. 4, April 2005, page A1, ABSTR. NR. 003, XP003004202
- BEERS M.H. ET AL.: 'LYMPHEDEMA' THE MERCK MANUAL OF DIAGNOSIS AND THERAPY, SEVENTEENTH EDITION 1999, page 1798, XP003004203
- KAJIYA K. ET AL.: 'Hepatocyte growth factor promotes lymphatic vessel formation and function' THE EMBO JOURNAL vol. 24, 17 August 2005, pages 2885 - 2895, XP002390312
- SZUBA ANDRZEJ ET AL: "Therapeutic lymphangiogenesis with human recombinant VEGF-C", FASEB JOURNAL, FED. OF AMERICAN SOC. FOR EXPERIMENTAL BIOLOGY, US, vol. 16, no. 14, 18 October 2002 (2002-10-18), pages 1-17, XP002441993, ISSN: 0892-6638, DOI: 10.1096/FJ.01-0901EDI
- YOON YOUNG-SUP ET AL: "VEGF-C gene therapy augments postnatal lymphangiogenesis and ameliorates secondary lymphedema", JOURNAL OF CLINICAL INVESTIGATION, AMERICAN SOCIETY FOR CLINICAL INVESTIGATION, US, vol. 111, no. 5, 1 March 2003 (2003-03-01) , pages 717-725, XP009170149, ISSN: 0021-9738
- GOLDMAN JEREMY ET AL: "Overexpression of VEGF-C causes transient lymphatic hyperplasia but not increased lymphangiogenesis in regenerating skin", CIRCULATION RESEARCH, GRUNE AND STRATTON, BALTIMORE, US, vol. 96, no. 11, 1 June 2005 (2005-06-01), pages 1193-1199, XP002454951, ISSN: 0009-7330, DOI: 10.1161/01.RES.0000168918.27576.78

### SEQUENCE LISTING

<110> ANGES MG, INC. OSAKA UNIVERSITY
<120> Lymphangiogenesis promoter
<130> MED-X0501Y1P
<150> JP 2005-219410
   <151> 2005-07-28
<150> JP 2006-148970
   <151> 2006-05-29
<160> 6
<170> PatentIn version 3.3
<210> 1
   <211> 2187
   <212> DNA
   <213> Homo sapiens
<400> 1
   atgtgggtga ccaaactcct gccagccctg ctgctgcagc atgtcctcct gcatctcctc     60
   ctgctcccca tcgccatccc ctatgcagag ggacaaagga aaagaagaaa tacaattcat    120
   gaattcaaaa aatcagcaaa gactacccta atcaaaatag atccagcact gaagataaaa    180
   accaaaaaag tgaatactgc agaccaatgt gctaatagat gtactaggaa taaaggactt    240
   ccattcactt gcaaggcttt tgtttttgat aaagcaagaa aacaatgcct ctggttcccc    300
   ttcaatagca tgtcaagtgg agtgaaaaaa gaatttggcc atgaatttga cctctatgaa    360
   aacaaagact acattagaaa ctgcatcatt ggtaaaggac gcagctacaa gggaacagta    420
   tctatcacta agagtggcat caaatgtcag ccctggagtt ccatgatacc acacgaacac    480
   agctttttgc cttcgagcta tcggggtaaa gacctacagg aaaactactg tcgaaatcct    540
   cgaggggaag aagggggacc ctggtgtttc acaagcaatc cagaggtacg ctacgaagtc    600
   tgtgacattc ctcagtgttc agaagttgaa tgcatgacct gcaatgggga gagttatcga 660
   ggtctcatgg atcatacaga atcaggcaag atttgtcagc gctgggatca tcagacacca 720
   caccggcaca aattcttgcc tgaaagatat cccgacaagg gctttgatga taattattgc 780
   cgcaatcccg atggccagcc gaggccatgg tgctatactc ttgaccctca cacccgctgg 840
   gagtactgtg caattaaaac atgcgctgac aatactatga atgacactga tgttcctttg 900
   gaaacaactg aatgcatcca aggtcaagga gaaggctaca ggggcactgt caataccatt 960
   tggaatggaa ttccatgtca gcgttgggat tctcagtatc ctcacgagca tgacatgact 1020
   cctgaaaatt tcaagtgcaa ggacctacga gaaaattact gccgaaatcc agatgggtct 1080
   gaatcaccct ggtgttttac cactgatcca aacatccgag ttggctactg ctcccaaatt 1140
   ccaaactgtg atatgtcaca tggacaagat tgttatcgtg ggaatggcaa aaattatatg 1200
   ggcaacttat cccaaacaag atctggacta acatgttcaa tgtgggacaa gaacatggaa 1260
   gacttacatc gtcatatctt ctgggaacca gatgcaagta agctgaatga gaattactgc 1320
   cgaaatccag atgatgatgc tcatggaccc tggtgctaca cgggaaatcc actcattcct 1380
   tgggattatt gccctatttc tcgttgtgaa ggtgatacca cacctacaat agtcaattta 1440
   gaccatcccg taatatcttg tgccaaaacg aaacaattgc gagttgtaaa tgggattcca 1500
   acacgaacaa acataggatg gatggttagt ttgagataca gaaataaaca tatctgcgga 1560
   ggatcattga taaaggagag ttgggttctt actgcacgac agtgtttccc ttctcgagac 1620
   ttgaaagatt atgaagcttg gcttggaatt catgatgtcc acggaagagg agatgagaaa 1680
   tgcaaacagg ttctcaatgt ttcccagctg gtatatggcc ctgaaggatc agatctggtt 1740
   ttaatgaagc ttgccaggcc tgctgtcctg gatgattttg ttagtacgat tgatttacct 1800
   aattatggat gcacaattcc tgaaaagacc agttgcagtg tttatggctg gggctacact 1860
   ggattgatca actatgatgg cctattacga gtggcacatc tctatataat gggaaatgag 1920
   aaatgcagcc agcatcatcg agggaaggtg actctgaatg agtctgaaat atgtgctggg 1980
   gctgaaaaga ttggatcagg accatgtgag ggggattatg gtggcccact tgtttgtgag 2040
   caacataaaa tgagaatggt tcttggtgtc attgttcctg gtcgtggatg tgccattcca 2100
   aatcgtcctg gtatttttgt ccgagtagca tattatgcaa aatggataca caaaattatt 2160
   ttaacatata aggtaccaca gtcatag 2187
<210> 2
   <211> 728
   <212> PRT
   <213> Homo sapiens
<400> 2
   Met Trp Val Thr Lys Leu Leu Pro Ala Leu Leu Leu Gln His Val Leu
   1 5 10 15
   Leu His Leu Leu Leu Leu Pro Ile Ala Ile Pro Tyr Ala Glu Gly Gln
   20 25 30
   Arg Lys Arg Arg Asn Thr Ile His Glu Phe Lys Lys Ser Ala Lys Thr
   35 40 45
   Thr Leu Ile Lys Ile Asp Pro Ala Leu Lys Ile Lys Thr Lys Lys Val
   50 55 60
   Asn Thr Ala Asp Gln Cys Ala Asn Arg Cys Thr Arg Asn Lys Gly Leu
   65 70 75 80
   Pro Phe Thr Cys Lys Ala Phe Val Phe Asp Lys Ala Arg Lys Gln Cys
   85 90 95
   Leu Trp Phe Pro Phe Asn Ser Met Ser Ser Gly Val Lys Lys Glu Phe
   100 105 110
   Gly His Glu Phe Asp Leu Tyr Glu Asn Lys Asp Tyr Ile Arg Asn Cys
   115 120 125
   Ile Ile Gly Lys Gly Arg Ser Tyr Lys Gly Thr Val Ser Ile Thr Lys
   130 135 140
   Ser Gly Ile Lys Cys Gln Pro Trp Ser Ser Met Ile Pro His Glu His
   145 150 155 160
   Ser Phe Leu Pro Ser Ser Tyr Arg Gly Lys Asp Leu Gln Glu Asn Tyr
   165 170 175
   Cys Arg Asn Pro Arg Gly Glu Glu Gly Gly Pro Trp Cys Phe Thr Ser
   180 185 190
   Asn Pro Glu Val Arg Tyr Glu Val Cys Asp Ile Pro Gln Cys Ser Glu
   195 200 205
   Val Glu Cys Met Thr Cys Asn Gly Glu Ser Tyr Arg Gly Leu Met Asp
   210 215 220
   His Thr Glu Ser Gly Lys Ile Cys Gln Arg Trp Asp His Gln Thr Pro
   225 230 235 240
   His Arg His Lys Phe Leu Pro Glu Arg Tyr Pro Asp Lys Gly Phe Asp
   245 250 255
   Asp Asn Tyr Cys Arg Asn Pro Asp Gly Gln Pro Arg Pro Trp Cys Tyr
   260 265 270
   Thr Leu Asp Pro His Thr Arg Trp Glu Tyr Cys Ala Ile Lys Thr Cys
   275 280 285
   Ala Asp Asn Thr Met Asn Asp Thr Asp Val Pro Leu Glu Thr Thr Glu
   290 295 300
   Cys Ile Gln Gly Gln Gly Glu Gly Tyr Arg Gly Thr Val Asn Thr Ile
   305 310 315 320
   Trp Asn Gly Ile Pro Cys Gln Arg Trp Asp Ser Gln Tyr Pro His Glu
   325 330 335
   His Asp Met Thr Pro Glu Asn Phe Lys Cys Lys Asp Leu Arg Glu Asn
   340 345 350
   Tyr Cys Arg Asn Pro Asp Gly Ser Glu Ser Pro Trp Cys Phe Thr Thr
   355 360 365
   Asp Pro Asn Ile Arg Val Gly Tyr Cys Ser Gln Ile Pro Asn Cys Asp
   370 375 380
   Met Ser His Gly Gln Asp Cys Tyr Arg Gly Asn Gly Lys Asn Tyr Met
   385 390 395 400
   Gly Asn Leu Ser Gln Thr Arg Ser Gly Leu Thr Cys Ser Met Trp Asp
   405 410 415
   Lys Asn Met Glu Asp Leu His Arg His Ile Phe Trp Glu Pro Asp Ala
   420 425 430
   Ser Lys Leu Asn Glu Asn Tyr Cys Arg Asn Pro Asp Asp Asp Ala His
   435 440 445
   Gly Pro Trp Cys Tyr Thr Gly Asn Pro Leu Ile Pro Trp Asp Tyr Cys
   450 455 460
   Pro Ile Ser Arg Cys Glu Gly Asp Thr Thr Pro Thr Ile Val Asn Leu
   465 470 475 480
   Asp His Pro Val Ile Ser Cys Ala Lys Thr Lys Gln Leu Arg Val Val
   485 490 495
   Asn Gly Ile Pro Thr Arg Thr Asn Ile Gly Trp Met Val Ser Leu Arg
   500 505 510
   Tyr Arg Asn Lys His Ile Cys Gly Gly Ser Leu Ile Lys Glu Ser Trp
   515 520 525
   Val Leu Thr Ala Arg Gln Cys Phe Pro Ser Arg Asp Leu Lys Asp Tyr
   530 535 540
   Glu Ala Trp Leu Gly Ile His Asp Val His Gly Arg Gly Asp Glu Lys
   545 550 555 560
   Cys Lys Gln Val Leu Asn Val Ser Gln Leu Val Tyr Gly Pro Glu Gly
   565 570 575
   Ser Asp Leu Val Leu Met Lys Leu Ala Arg Pro Ala Val Leu Asp Asp
   580 585 590
   Phe Val Ser Thr Ile Asp Leu Pro Asn Tyr Gly Cys Thr Ile Pro Glu
   595 600 605
   Lys Thr Ser Cys Ser Val Tyr Gly Trp Gly Tyr Thr Gly Leu Ile Asn
   610 615 620
   Tyr Asp Gly Leu Leu Arg Val Ala His Leu Tyr Ile Met Gly Asn Glu
   625 630 635 640
   Lys Cys Ser Gln His His Arg Gly Lys Val Thr Leu Asn Glu Ser Glu
   645 650 655
   Ile Cys Ala Gly Ala Glu Lys Ile Gly Ser Gly Pro Cys Glu Gly Asp
   660 665 670
   Tyr Gly Gly Pro Leu Val Cys Glu Gln His Lys Met Arg Met Val Leu
   675 680 685
   Gly Val Ile Val Pro Gly Arg Gly Cys Ala Ile Pro Asn Arg Pro Gly
   690 695 700
   Ile Phe Val Arg Val Ala Tyr Tyr Ala Lys Trp Ile His Lys Ile Ile
   705 710 715 720
   Leu Thr Tyr Lys Val Pro Gln Ser
   725
<210> 3
   <211> 4173
   <212> DNA
   <213> Homo sapiens
<400> 3
   atgaaggccc ccgctgtgct tgcacctggc atcctcgtgc tcctgtttac cttggtgcag 60
   aggagcaatg gggagtgtaa agaggcacta gcaaagtccg agatgaatgt gaatatgaag 120
   tatcagcttc ccaacttcac cgcggaaaca cccatccaga atgtcattct acatgagcat 180
   cacattttcc ttggtgccac taactacatt tatgttttaa atgaggaaga ccttcagaag 240
   gttgctgagt acaagactgg gcctgtgctg gaacacccag attgtttccc atgtcaggac 300
   tgcagcagca aagccaattt atcaggaggt gtttggaaag ataacatcaa catggctcta 360
   gttgtcgaca cctactatga tgatcaactc attagctgtg gcagcgtcaa cagagggacc 420
   tgccagcgac atgtctttcc ccacaatcat actgctgaca tacagtcgga ggttcactgc 480
   atattctccc cacagataga agagcccagc cagtgtcctg actgtgtggt gagcgccctg 540
   ggagccaaag tcctttcatc tgtaaaggac cggttcatca acttctttgt aggcaatacc 600
   ataaattctt cttatttccc agatcatcca ttgcattcga tatcagtgag aaggctaaag 660
   gaaacgaaag atggttttat gtttttgacg gaccagtcct acattgatgt tttacctgag 720
   ttcagagatt cttaccccat taagtatgtc catgcctttg aaagcaacaa ttttatttac 780
   ttcttgacgg tccaaaggga aactctagat gctcagactt ttcacacaag aataatcagg 840
   ttctgttcca taaactctgg attgcattcc tacatggaaa tgcctctgga gtgtattctc 900
   acagaaaaga gaaaaaagag atccacaaag aaggaagtgt ttaatatact tcaggctgcg 960
   tatgtcagca agcctggggc ccagcttgct agacaaatag gagccagcct gaatgatgac 1020
   attcttttcg gggtgttcgc acaaagcaag ccagattctg ccgaaccaat ggatcgatct 1080
   gccatgtgtg cattccctat caaatatgtc aacgacttct tcaacaagat cgtcaacaaa 1140
   aacaatgtga gatgtctcca gcatttttac ggacccaatc atgagcactg ctttaatagg 1200
   acacttctga gaaattcatc aggctgtgaa gcgcgccgtg atgaatatcg aacagagttt 1260
   accacagctt tgcagcgcgt tgacttattc atgggtcaat tcagcgaagt cctcttaaca 1320
   tctatatcca ccttcattaa aggagacctc accatagcta atcttgggac atcagagggt 1380
   cgcttcatgc aggttgtggt ttctcgatca ggaccatcaa cccctcatgt gaattttctc 1440
   ctggactccc atccagtgtc tccagaagtg attgtggagc atacattaaa ccaaaatggc 1500
   tacacactgg ttatcactgg gaagaagatc acgaagatcc cattgaatgg cttgggctgc 1560
   agacatttcc agtcctgcag tcaatgcctc tctgccccac cctttgttca gtgtggctgg 1620
   tgccacgaca aatgtgtgcg atcggaggaa tgcctgagcg ggacatggac tcaacagatc 1680
   tgtctgcctg caatctacaa ggttttccca aatagtgcac cccttgaagg agggacaagg 1740
   ctgaccatat gtggctggga ctttggattt cggaggaata ataaatttga tttaaagaaa 1800
   actagagttc tccttggaaa tgagagctgc accttgactt taagtgagag cacgatgaat 1860
   acattgaaat gcacagttgg tcctgccatg aataagcatt tcaatatgtc cataattatt 1920
   tcaaatggcc acgggacaac acaatacagt acattctcct atgtggatcc tgtaataaca 1980
   agtatttcgc cgaaatacgg tcctatggct ggtggcactt tacttacttt aactggaaat 2040
   tacctaaaca gtgggaattc tagacacatt tcaattggtg gaaaaacatg tactttaaaa 2100
   agtgtgtcaa acagtattct tgaatgttat accccagccc aaaccatttc aactgagttt 2160
   gctgttaaat tgaaaattga cttagccaac cgagagacaa gcatcttcag ttaccgtgaa 2220
   gatcccattg tctatgaaat tcatccaacc aaatctttta ttagtggtgg gagcacaata 2280
   acaggtgttg ggaaaaacct gaattcagtt agtgtcccga gaatggtcat aaatgtgcat 2340
   gaagcaggaa ggaactttac agtggcatgt caacatcgct ctaattcaga gataatctgt 2400
   tgtaccactc cttccctgca acagctgaat ctgcaactcc ccctgaaaac caaagccttt 2460
   ttcatgttag atgggatcct ttccaaatac tttgatctca tttatgtaca taatcctgtg 2520
   tttaagcctt ttgaaaagcc agtgatgatc tcaatgggca atgaaaatgt actggaaatt 2580
   aagggaaatg atattgaccc tgaagcagtt aaaggtgaag tgttaaaagt tggaaataag 2640
   agctgtgaga atatacactt acattctgaa gccgttttat gcacggtccc caatgacctg 2700
   ctgaaattga acagcgagct aaatatagag tggaagcaag caatttcttc aaccgtcctt 2760
   ggaaaagtaa tagttcaacc agatcagaat ttcacaggat tgattgctgg tgttgtctca 2820
   atatcaacag cactgttatt actacttggg tttttcctgt ggctgaaaaa gagaaagcaa 2880
   attaaagatc tgggcagtga attagttcgc tacgatgcaa gagtacacac tcctcatttg 2940
   gataggcttg taagtgcccg aagtgtaagc ccaactacag aaatggtttc aaatgaatct 3000
   gtagactacc gagctacttt tccagaagat cagtttccta attcatctca gaacggttca 3060
   tgccgacaag tgcagtatcc tctgacagac atgtccccca tcctaactag tggggactct 3120
   gatatatcca gtccattact gcaaaatact gtccacattg acctcagtgc tctaaatcca 3180
   gagctggtcc aggcagtgca gcatgtagtg attgggccca gtagcctgat tgtgcatttc 3240
   aatgaagtca taggaagagg gcattttggt tgtgtatatc atgggacttt gttggacaat 3300
   gatggcaaga aaattcactg tgctgtgaaa tccttgaaca gaatcactga cataggagaa 3360
   gtttcccaat ttctgaccga gggaatcatc atgaaagatt ttagtcatcc caatgtcctc 3420
   tcgctcctgg gaatctgcct gcgaagtgaa gggtctccgc tggtggtcct accatacatg 3480
   aaacatggag atcttcgaaa tttcattcga aatgagactc ataatccaac tgtaaaagat 3540
   cttattggct ttggtcttca agtagccaaa ggcatgaaat atcttgcaag caaaaagttt 3600
   gtccacagag acttggctgc aagaaactgt atgctggatg aaaaattcac agtcaaggtt 3660
   gctgattttg gtcttgccag agacatgtat gataaagaat actatagtgt acacaacaaa 3720
   acaggtgcaa agctgccagt gaagtggatg gctttggaaa gtctgcaaac tcaaaagttt 3780
   accaccaagt cagatgtgtg gtcctttggc gtgctcctct gggagctgat gacaagagga 3840
   gccccacctt atcctgacgt aaacaccttt gatataactg tttacttgtt gcaagggaga 3900
   agactcctac aacccgaata ctgcccagac cccttatatg aagtaatgct aaaatgctgg 3960
   caccctaaag ccgaaatgcg cccatccttt tctgaactgg tgtcccggat atcagcgatc 4020
   ttctctactt tcattgggga gcactatgtc catgtgaacg ctacttatgt gaacgtaaaa 4080
   tgtgtcgctc cgtatccttc tctgttgtca tcagaagata acgctgatga tgaggtggac 4140
   acacgaccag cctccttctg ggagacatca tag 4173
<210> 4
   <211> 1390
   <212> PRT
   <213> Homo sapiens
<400> 4
   Met Lys Ala Pro Ala Val Leu Ala Pro Gly Ile Leu Val Leu Leu Phe
   1 5 10 15
   Thr Leu Val Gln Arg Ser Asn Gly Glu Cys Lys Glu Ala Leu Ala Lys
   20 25 30
   Ser Glu Met Asn Val Asn Met Lys Tyr Gln Leu Pro Asn Phe Thr Ala
   35 40 45
   Glu Thr Pro Ile Gln Asn Val Ile Leu His Glu His His Ile Phe Leu
   50 55 60
   Gly Ala Thr Asn Tyr Ile Tyr Val Leu Asn Glu Glu Asp Leu Gln Lys
   65 70 75 80
   Val Ala Glu Tyr Lys Thr Gly Pro Val Leu Glu His Pro Asp Cys Phe
   85 90 95
   Pro Cys Gln Asp Cys Ser Ser Lys Ala Asn Leu Ser Gly Gly Val Trp
   100 105 110
   Lys Asp Asn Ile Asn Met Ala Leu Val Val Asp Thr Tyr Tyr Asp Asp
   115 120 125
   Gln Leu Ile Ser Cys Gly Ser Val Asn Arg Gly Thr Cys Gln Arg His
   130 135 140
   Val Phe Pro His Asn His Thr Ala Asp Ile Gln Ser Glu Val His Cys
   145 150 155 160
   Ile Phe Ser Pro Gln Ile Glu Glu Pro Ser Gln Cys Pro Asp Cys Val
   165 170 175
   Val Ser Ala Leu Gly Ala Lys Val Leu Ser Ser Val Lys Asp Arg Phe
   180 185 190
   Ile Asn Phe Phe Val Gly Asn Thr Ile Asn Ser Ser Tyr Phe Pro Asp
   195 200 205
   His Pro Leu His Ser Ile Ser Val Arg Arg Leu Lys Glu Thr Lys Asp
   210 215 220
   Gly Phe Met Phe Leu Thr Asp Gln Ser Tyr Ile Asp Val Leu Pro Glu
   225 230 235 240
   Phe Arg Asp Ser Tyr Pro Ile Lys Tyr Val His Ala Phe Glu Ser Asn
   245 250 255
   Asn Phe Ile Tyr Phe Leu Thr Val Gln Arg Glu Thr Leu Asp Ala Gln
   260 265 270
   Thr Phe His Thr Arg Ile Ile Arg Phe Cys Ser Ile Asn Ser Gly Leu
   275 280 285
   His Ser Tyr Met Glu Met Pro Leu Glu Cys Ile Leu Thr Glu Lys Arg
   290 295 300
   Lys Lys Arg Ser Thr Lys Lys Glu Val Phe Asn Ile Leu Gln Ala Ala
   305 310 315 320
   Tyr Val Ser Lys Pro Gly Ala Gln Leu Ala Arg Gln Ile Gly Ala Ser
   325 330 335
   Leu Asn Asp Asp Ile Leu Phe Gly Val Phe Ala Gln Ser Lys Pro Asp
   340 345 350
   Ser Ala Glu Pro Met Asp Arg Ser Ala Met Cys Ala Phe Pro Ile Lys
   355 360 365
   Tyr Val Asn Asp Phe Phe Asn Lys Ile Val Asn Lys Asn Asn Val Arg
   370 375 380
   Cys Leu Gln His Phe Tyr Gly Pro Asn His Glu His Cys Phe Asn Arg
   385 390 395 400
   Thr Leu Leu Arg Asn Ser Ser Gly Cys Glu Ala Arg Arg Asp Glu Tyr
   405 410 415
   Arg Thr Glu Phe Thr Thr Ala Leu Gln Arg Val Asp Leu Phe Met Gly
   420 425 430
   Gln Phe Ser Glu Val Leu Leu Thr Ser Ile Ser Thr Phe Ile Lys Gly
   435 440 445
   Asp Leu Thr Ile Ala Asn Leu Gly Thr Ser Glu Gly Arg Phe Met Gln
   450 455 460
   Val Val Val Ser Arg Ser Gly Pro Ser Thr Pro His Val Asn Phe Leu
   465 470 475 480
   Leu Asp Ser His Pro Val Ser Pro Glu Val Ile Val Glu His Thr Leu
   485 490 495
   Asn Gln Asn Gly Tyr Thr Leu Val Ile Thr Gly Lys Lys Ile Thr Lys
   500 505 510
   Ile Pro Leu Asn Gly Leu Gly Cys Arg His Phe Gln Ser Cys Ser Gln
   515 520 525
   Cys Leu Ser Ala Pro Pro Phe Val Gln Cys Gly Trp Cys His Asp Lys
   530 535 540
   Cys Val Arg Ser Glu Glu Cys Leu Ser Gly Thr Trp Thr Gln Gln Ile
   545 550 555 560
   Cys Leu Pro Ala Ile Tyr Lys Val Phe Pro Asn Ser Ala Pro Leu Glu
   565 570 575
   Gly Gly Thr Arg Leu Thr Ile Cys Gly Trp Asp Phe Gly Phe Arg Arg
   580 585 590
   Asn Asn Lys Phe Asp Leu Lys Lys Thr Arg Val Leu Leu Gly Asn Glu
   595 600 605
   Ser Cys Thr Leu Thr Leu Ser Glu Ser Thr Met Asn Thr Leu Lys Cys
   610 615 620
   Thr Val Gly Pro Ala Met Asn Lys His Phe Asn Met Ser Ile Ile Ile
   625 630 635 640
   Ser Asn Gly His Gly Thr Thr Gln Tyr Ser Thr Phe Ser Tyr Val Asp
   645 650 655
   Pro Val Ile Thr Ser Ile Ser Pro Lys Tyr Gly Pro Met Ala Gly Gly
   660 665 670
   Thr Leu Leu Thr Leu Thr Gly Asn Tyr Leu Asn Ser Gly Asn Ser Arg
   675 680 685
   His Ile Ser Ile Gly Gly Lys Thr Cys Thr Leu Lys Ser Val Ser Asn
   690 695 700
   Ser Ile Leu Glu Cys Tyr Thr Pro Ala Gln Thr Ile Ser Thr Glu Phe
   705 710 715 720
   Ala Val Lys Leu Lys Ile Asp Leu Ala Asn Arg Glu Thr Ser Ile Phe
   725 730 735
   Ser Tyr Arg Glu Asp Pro Ile Val Tyr Glu Ile His Pro Thr Lys Ser
   740 745 750
   Phe Ile Ser Gly Gly Ser Thr Ile Thr Gly Val Gly Lys Asn Leu Asn
   755 760 765
   Ser Val Ser Val Pro Arg Met Val Ile Asn Val His Glu Ala Gly Arg
   770 775 780
   Asn Phe Thr Val Ala Cys Gln His Arg Ser Asn Ser Glu Ile Ile Cys
   785 790 795 800
   Cys Thr Thr Pro Ser Leu Gln Gln Leu Asn Leu Gln Leu Pro Leu Lys
   805 810 815
   Thr Lys Ala Phe Phe Met Leu Asp Gly Ile Leu Ser Lys Tyr Phe Asp
   820 825 830
   Leu Ile Tyr Val His Asn Pro Val Phe Lys Pro Phe Glu Lys Pro Val
   835 840 845
   Met Ile Ser Met Gly Asn Glu Asn Val Leu Glu Ile Lys Gly Asn Asp
   850 855 860
   Ile Asp Pro Glu Ala Val Lys Gly Glu Val Leu Lys Val Gly Asn Lys
   865 870 875 880
   Ser Cys Glu Asn Ile His Leu His Ser Glu Ala Val Leu Cys Thr Val
   885 890 895
   Pro Asn Asp Leu Leu Lys Leu Asn Ser Glu Leu Asn Ile Glu Trp Lys
   900 905 910
   Gln Ala Ile Ser Ser Thr Val Leu Gly Lys Val Ile Val Gln Pro Asp
   915 920 925
   Gln Asn Phe Thr Gly Leu Ile Ala Gly Val Val Ser Ile Ser Thr Ala
   930 935 940
   Leu Leu Leu Leu Leu Gly Phe Phe Leu Trp Leu Lys Lys Arg Lys Gln
   945 950 955 960
   Ile Lys Asp Leu Gly Ser Glu Leu Val Arg Tyr Asp Ala Arg Val His
   965 970 975
   Thr Pro His Leu Asp Arg Leu Val Ser Ala Arg Ser Val Ser Pro Thr
   980 985 990
   Thr Glu Met Val Ser Asn Glu Ser Val Asp Tyr Arg Ala Thr Phe Pro
   995 1000 1005
   Glu Asp Gln Phe Pro Asn Ser Ser Gln Asn Gly Ser Cys Arg Gln
   1010 1015 1020
   Val Gln Tyr Pro Leu Thr Asp Met Ser Pro Ile Leu Thr Ser Gly
   1025 1030 1035
   Asp Ser Asp Ile Ser Ser Pro Leu Leu Gln Asn Thr Val His Ile
   1040 1045 1050
   Asp Leu Ser Ala Leu Asn Pro Glu Leu Val Gln Ala Val Gln His
   1055 1060 1065
   Val Val Ile Gly Pro Ser Ser Leu Ile Val His Phe Asn Glu Val
   1070 1075 1080
   Ile Gly Arg Gly His Phe Gly Cys Val Tyr His Gly Thr Leu Leu
   1085 1090 1095
   Asp Asn Asp Gly Lys Lys Ile His Cys Ala Val Lys Ser Leu Asn
   1100 1105 1110
   Arg Ile Thr Asp Ile Gly Glu Val Ser Gln Phe Leu Thr Glu Gly
   1115 1120 1125
   Ile Ile Met Lys Asp Phe Ser His Pro Asn Val Leu Ser Leu Leu
   1130 1135 1140
   Gly Ile Cys Leu Arg Ser Glu Gly Ser Pro Leu Val Val Leu Pro
   1145 1150 1155
   Tyr Met Lys His Gly Asp Leu Arg Asn Phe Ile Arg Asn Glu Thr
   1160 1165 1170
   His Asn Pro Thr Val Lys Asp Leu Ile Gly Phe Gly Leu Gln Val
   1175 1180 1185
   Ala Lys Gly Met Lys Tyr Leu Ala Ser Lys Lys Phe Val His Arg
   1190 1195 1200
   Asp Leu Ala Ala Arg Asn Cys Met Leu Asp Glu Lys Phe Thr Val
   1205 1210 1215
   Lys Val Ala Asp Phe Gly Leu Ala Arg Asp Met Tyr Asp Lys Glu
   1220 1225 1230
   Tyr Tyr Ser Val His Asn Lys Thr Gly Ala Lys Leu Pro Val Lys
   1235 1240 1245
   Trp Met Ala Leu Glu Ser Leu Gln Thr Gln Lys Phe Thr Thr Lys
   1250 1255 1260
   Ser Asp Val Trp Ser Phe Gly Val Leu Leu Trp Glu Leu Met Thr
   1265 1270 1275
   Arg Gly Ala Pro Pro Tyr Pro Asp Val Asn Thr Phe Asp Ile Thr
   1280 1285 1290
   Val Tyr Leu Leu Gln Gly Arg Arg Leu Leu Gln Pro Glu Tyr Cys
   1295 1300 1305
   Pro Asp Pro Leu Tyr Glu Val Met Leu Lys Cys Trp His Pro Lys
   1310 1315 1320
   Ala Glu Met Arg Pro Ser Phe Ser Glu Leu Val Ser Arg Ile Ser
   1325 1330 1335
   Ala Ile Phe Ser Thr Phe Ile Gly Glu His Tyr Val His Val Asn
   1340 1345 1350
   Ala Thr Tyr Val Asn Val Lys Cys Val Ala Pro Tyr Pro Ser Leu
   1355 1360 1365
   Leu Ser Ser Glu Asp Asn Ala Asp Asp Glu Val Asp Thr Arg Pro
   1370 1375 1380
   Ala Ser Phe Trp Glu Thr Ser
   1385 1390
<210> 5
   <211> 5181
   <212> DNA
   <213> Artificial
<220>
   <223> Description of Artificial Sequence: pVAX1HGF/MGB1
<400> 5
   gctgcttcgc gatgtacggg ccagatatac gcgttgacat tgattattga ctagttatta 60
   atagtaatca attacggggt cattagttca tagcccatat atggagttcc gcgttacata 120
   acttacggta aatggcccgc ctggctgacc gcccaacgac ccccgcccat tgacgtcaat 180
   aatgacgtat gttcccatag taacgccaat agggactttc cattgacgtc aatgggtgga 240
   gtatttacgg taaactgccc acttggcagt acatcaagtg tatcatatgc caagtacgcc 300
   ccctattgac gtcaatgacg gtaaatggcc cgcctggcat tatgcccagt acatgacctt 360
   atgggacttt cctacttggc agtacatcta cgtattagtc atcgctatta ccatggtgat 420
   gcggttttgg cagtacatca atgggcgtgg atagcggttt gactcacggg gatttccaag 480
   tctccacccc attgacgtca atgggagttt gttttggcac caaaatcaac gggactttcc 540
   aaaatgtcgt aacaactccg ccccattgac gcaaatgggc ggtaggcgtg tacggtggga 600
   ggtctatata agcagagctc tctggctaac tagagaaccc actgcttact ggcttatcga 660
   aattaatacg actcactata gggagaccca agctggctag cgtttaaact taagcttggt 720
   accgagctcg gatccgccag cccgtccagc agcaccatgt gggtgaccaa actcctgcca 780
   gccctgctgc tgcagcatgt cctcctgcat ctcctcctgc tccccatcgc catcccctat 840
   gcagagggac aaaggaaaag aagaaataca attcatgaat tcaaaaaatc agcaaagact 900
   accctaatca aaatagatcc agcactgaag ataaaaacca aaaaagtgaa tactgcagac 960
   caatgtgcta atagatgtac taggaataaa ggacttccat tcacttgcaa ggcttttgtt 1020
   tttgataaag caagaaaaca atgcctctgg ttccccttca atagcatgtc aagtggagtg 1080
   aaaaaagaat ttggccatga atttgacctc tatgaaaaca aagactacat tagaaactgc 1140
   atcattggta aaggacgcag ctacaaggga acagtatcta tcactaagag tggcatcaaa 1200
   tgtcagccct ggagttccat gataccacac gaacacagct ttttgccttc gagctatcgg 1260
   ggtaaagacc tacaggaaaa ctactgtcga aatcctcgag gggaagaagg gggaccctgg 1320
   tgtttcacaa gcaatccaga ggtacgctac gaagtctgtg acattcctca gtgttcagaa 1380
   gttgaatgca tgacctgcaa tggggagagt tatcgaggtc tcatggatca tacagaatca 1440
   ggcaagattt gtcagcgctg ggatcatcag acaccacacc ggcacaaatt cttgcctgaa 1500
   agatatcccg acaagggctt tgatgataat tattgccgca atcccgatgg ccagccgagg 1560
   ccatggtgct atactcttga ccctcacacc cgctgggagt actgtgcaat taaaacatgc 1620
   gctgacaata ctatgaatga cactgatgtt cctttggaaa caactgaatg catccaaggt 1680
   caaggagaag gctacagggg cactgtcaat accatttgga atggaattcc atgtcagcgt 1740
   tgggattctc agtatcctca cgagcatgac atgactcctg aaaatttcaa gtgcaaggac 1800
   ctacgagaaa attactgccg aaatccagat gggtctgaat caccctggtg ttttaccact 1860
   gatccaaaca tccgagttgg ctactgctcc caaattccaa actgtgatat gtcacatgga 1920
   caagattgtt atcgtgggaa tggcaaaaat tatatgggca acttatccca aacaagatct 1980
   ggactaacat gttcaatgtg ggacaagaac atggaagact tacatcgtca tatcttctgg 2040
   gaaccagatg caagtaagct gaatgagaat tactgccgaa atccagatga tgatgctcat 2100
   ggaccctggt gctacacggg aaatccactc attccttggg attattgccc tatttctcgt 2160
   tgtgaaggtg ataccacacc tacaatagtc aatttagacc atcccgtaat atcttgtgcc 2220
   aaaacgaaac aattgcgagt tgtaaatggg attccaacac gaacaaacat aggatggatg 2280
   gttagtttga gatacagaaa taaacatatc tgcggaggat cattgataaa ggagagttgg 2340
   gttcttactg cacgacagtg tttcccttct cgagacttga aagattatga agcttggctt 2400
   ggaattcatg atgtccacgg aagaggagat gagaaatgca aacaggttct caatgtttcc 2460
   cagctggtat atggccctga aggatcagat ctggttttaa tgaagcttgc caggcctgct 2520
   gtcctggatg attttgttag tacgattgat ttacctaatt atggatgcac aattcctgaa 2580
   aagaccagtt gcagtgttta tggctggggc tacactggat tgatcaacta tgatggccta 2640
   ttacgagtgg cacatctcta tataatggga aatgagaaat gcagccagca tcatcgaggg 2700
   aaggtgactc tgaatgagtc tgaaatatgt gctggggctg aaaagattgg atcaggacca 2760
   tgtgaggggg attatggtgg cccacttgtt tgtgagcaac ataaaatgag aatggttctt 2820
   ggtgtcattg ttcctggtcg tggatgtgcc attccaaatc gtcctggtat ttttgtccga 2880
   gtagcatatt atgcaaaatg gatacacaaa attattttaa catataaggt accacagtca 2940
   tagctgttaa cccgggtcga agcggccgct cgagtctaga gggcccgttt aaacccgctg 3000
   atcagcctcg actgtgcctt ctagttgcca gccatctgtt gtttgcccct cccccgtgcc 3060
   ttccttgacc ctggaaggtg ccactcccac tgtcctttcc taataaaatg aggaaattgc 3120
   atcgcattgt ctgagtaggt gtcattctat tctggggggt ggggtggggc aggacagcaa 3180
   gggggaggat tgggaagaca atagcaggca tgctggggat gcggtgggct ctatggcttc 3240
   tactgggcgg ttttatggac agcaagcgaa ccggaattgc cagctggggc gccctctggt 3300
   aaggttggga agccctgcaa agtaaactgg atggctttct tgccgccaag gatctgatgg 3360
   cgcaggggat caagctctga tcaagagaca ggatgaggat cgtttcgcat gattgaacaa 3420
   gatggattgc acgcaggttc tccggccgct tgggtggaga ggctattcgg ctatgactgg 3480
   gcacaacaga caatcggctg ctctgatgcc gccgtgttcc ggctgtcagc gcaggggcgc 3540
   ccggttcttt ttgtcaagac cgacctgtcc ggtgccctga atgaactgca agacgaggca 3600
   gcgcggctat cgtggctggc cacgacgggc gttccttgcg cagctgtgct cgacgttgtc 3660
   actgaagcgg gaagggactg gctgctattg ggcgaagtgc cggggcagga tctcctgtca 3720
   tctcaccttg ctcctgccga gaaagtatcc atcatggctg atgcaatgcg gcggctgcat 3780
   acgcttgatc cggctacctg cccattcgac caccaagcga aacatcgcat cgagcgagca 3840
   cgtactcgga tggaagccgg tcttgtcgat caggatgatc tggacgaaga gcatcagggg 3900
   ctcgcgccag ccgaactgtt cgccaggctc aaggcgagca tgcccgacgg cgaggatctc 3960
   gtcgtgaccc atggcgatgc ctgcttgccg aatatcatgg tggaaaatgg ccgcttttct 4020
   ggattcatcg actgtggccg gctgggtgtg gcggaccgct atcaggacat agcgttggct 4080
   acccgtgata ttgctgaaga gcttggcggc gaatgggctg accgcttcct cgtgctttac 4140
   ggtatcgccg ctcccgattc gcagcgcatc gccttctatc gccttcttga cgagttcttc 4200
   tgaattatta acgcttacaa tttcctgatg cggtattttc tccttacgca tctgtgcggt 4260
   atttcacacc gcatcaggtg gcacttttcg gggaaatgtg cgcggaaccc ctatttgttt 4320
   atttttctaa atacattcaa atatgtatcc gctcatgaga caataaccct gataaatgct 4380
   tcaataatag cacgtgctaa aacttcattt ttaatttaaa aggatctagg tgaagatcct 4440
   ttttgataat ctcatgacca aaatccctta acgtgagttt tcgttccact gagcgtcaga 4500
   ccccgtagaa aagatcaaag gatcttcttg agatcctttt tttctgcgcg taatctgctg 4560
   cttgcaaaca aaaaaaccac cgctaccagc ggtggtttgt ttgccggatc aagagctacc 4620
   aactcttttt ccgaaggtaa ctggcttcag cagagcgcag ataccaaata ctgttcttct 4680
   agtgtagccg tagttaggcc accacttcaa gaactctgta gcaccgccta catacctcgc 4740
   tctgctaatc ctgttaccag tggctgctgc cagtggcgat aagtcgtgtc ttaccgggtt 4800
   ggactcaaga cgatagttac cggataaggc gcagcggtcg ggctgaacgg ggggttcgtg 4860
   cacacagccc agcttggagc gaacgaccta caccgaactg agatacctac agcgtgagct 4920
   atgagaaagc gccacgcttc ccgaagggag aaaggcggac aggtatccgg taagcggcag 4980
   ggtcggaaca ggagagcgca cgagggagct tccaggggga aacgcctggt atctttatag 5040
   tcctgtcggg tttcgccacc tctgacttga gcgtcgattt ttgtgatgct cgtcaggggg 5100
   gcggagccta tggaaaaacg ccagcaacgc ggccttttta cggttcctgg ccttttgctg 5160
   gccttttgct cacatgttct t 5181
<210> 6
   <211> 7681
   <212> DNA
   <213> Artificial
<220>
   <223> Description of Artificial Sequence: pcDNA3.1(-)HGF Sequence
<400> 6
   gacggatcgg gagatctccc gatcccctat ggtgcactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggag tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gctggctagc    900
   gtttaaacgg gccctctaga ctcgagcggc cgctctagaa ctagctggat cctgccagcc    960
   cgtccagcag caccatgtgg gtgaccaaac tcctgccagc cctgctgctg cagcatgtcc   1020
   tcctgcatct cctcctgctc cccatcgcca tcccctatgc agagggacaa aggaaaagaa   1080
   gaaatacaat tcatgaattc aaaaaatcag caaagactac cctaatcaaa atagatccag   1140
   cactgaagat aaaaaccaaa aaagtgaata ctgcagacca atgtgctaat agatgtacta   1200
   ggaataaagg acttccattc acttgcaagg cttttgtttt tgataaagca agaaaacaat   1260
   gcctctggtt ccccttcaat agcatgtcaa gtggagtgaa aaaagaattt ggccatgaat 1320
   ttgacctcta tgaaaacaaa gactacatta gaaactgcat cattggtaaa ggacgcagct 1380
   acaagggaac agtatctatc actaagagtg gcatcaaatg tcagccctgg agttccatga 1440
   taccacacga acacagcttt ttgccttcga gctatcgggg taaagaccta caggaaaact 1500
   actgtcgaaa tcctcgaggg gaagaagggg gaccctggtg tttcacaagc aatccagagg 1560
   tacgctacga agtctgtgac attcctcagt gttcagaagt tgaatgcatg acctgcaatg 1620
   gggagagtta tcgaggtctc atggatcata cagaatcagg caagatttgt cagcgctggg 1680
   atcatcagac accacaccgg cacaaattct tgcctgaaag atatcccgac aagggctttg 1740
   atgataatta ttgccgcaat cccgatggcc agccgaggcc atggtgctat actcttgacc 1800
   ctcacacccg ctgggagtac tgtgcaatta aaacatgcgc tgacaatact atgaatgaca 1860
   ctgatgttcc tttggaaaca actgaatgca tccaaggtca aggagaaggc tacaggggca 1920
   ctgtcaatac catttggaat ggaattccat gtcagcgttg ggattctcag tatcctcacg 1980
   agcatgacat gactcctgaa aatttcaagt gcaaggacct acgagaaaat tactgccgaa 2040
   atccagatgg gtctgaatca ccctggtgtt ttaccactga tccaaacatc cgagttggct 2100
   actgctccca aattccaaac tgtgatatgt cacatggaca agattgttat cgtgggaatg 2160
   gcaaaaatta tatgggcaac ttatcccaaa caagatctgg actaacatgt tcaatgtggg 2220
   acaagaacat ggaagactta catcgtcata tcttctggga accagatgca agtaagctga 2280
   atgagaatta ctgccgaaat ccagatgatg atgctcatgg accctggtgc tacacgggaa 2340
   atccactcat tccttgggat tattgcccta tttctcgttg tgaaggtgat accacaccta 2400
   caatagtcaa tttagaccat cccgtaatat cttgtgccaa aacgaaacaa ttgcgagttg 2460
   taaatgggat tccaacacga acaaacatag gatggatggt tagtttgaga tacagaaata 2520
   aacatatctg cggaggatca ttgataaagg agagttgggt tcttactgca cgacagtgtt 2580
   tcccttctcg agacttgaaa gattatgaag cttggcttgg aattcatgat gtccacggaa 2640
   gaggagatga gaaatgcaaa caggttctca atgttttcca gctggtatat ggccctgaag 2700
   gatcagatct ggttttaatg aagcttgcca ggcctgctgt cdtggatgat tttgttagta 2760
   cgattgattt acctaattat ggatgcacaa ttcctgaaaa gaccagttgc agtgtttatg 2820
   gctggggcta cactggattg atcaactatg atggcctatt acgagtggca catctctata 2880
   taatgggaaa tgagaaatgc agccagcatc atcgagggaa ggtgactctg aatgagtctg 2940
   aaatatgtgc tggggctgaa aagattggat caggaccatg tgagggggat tatggtggcc 3000
   cacttgtttg tgagcaacat aaaatgagaa tggttcttgg tgtcattgtt cctggtcgtg 3060
   gatgtgccat tccaaatcgt cctggtattt ttgtccgagt agcatattat gcaaaatgga 3120
   tacacaaaat tattttaaca tataaggtac cacagtcata gctgttaacc cgggtcgaag 3180
   cggccgccac tgtgctggat atctgcagaa ttccaccaca ctggactagt ggatccgagc 3240
   tcggtaccaa gcttaagttt aaaccgctga tcagcctcga ctgtgccttc tagttgccag 3300
   ccatctgttg tttgcccctc ccccgtgcct tccttgaccc tggaaggtgc cactcccact 3360
   gtcctttcct aataaaatga ggaaattgca tcgcattgtc tgagtaggtg tcattctatt 3420
   ctggggggtg gggtggggca ggacagcaag ggggaggatt gggaagacaa tagcaggcat 3480
   gctggggatg cggtgggctc tatggcttct gaggcggaaa gaaccagctg gggctctagg 3540
   gggtatcccc acgcgccctg tagcggcgca ttaagcgcgg cgggtgtggt ggttacgcgc 3600
   agcgtgaccg ctacacttgc cagcgcccta gcgcccgctc ctttcgcttt cttcccttcc 3660
   tttctcgcca cgttcgccgg ctttccccgt caagctctaa atcgggggct ccctttaggg 3720
   ttccgattta gtgctttacg gcacctcgac cccaaaaaac ttgattaggg tgatggttca 3780
   cgtagtgggc catcgccctg atagacggtt tttcgccctt tgacgttgga gtccacgttc 3840
   tttaatagtg gactcttgtt ccaaactgga acaacactca accctatctc ggtctattct 3900
   tttgatttat aagggatttt gccgatttcg gcctattggt taaaaaatga gctgatttaa 3960
   caaaaattta acgcgaatta attctgtgga atgtgtgtca gttagggtgt ggaaagtccc 4020
   caggctcccc agcaggcaga agtatgcaaa gcatgcatct caattagtca gcaaccaggt 4080
   gtggaaagtc cccaggctcc ccagcaggca gaagtatgca aagcatgcat ctcaattagt 4140
   cagcaaccat agtcccgccc ctaactccgc ccatcccgcc cctaactccg cccagttccg 4200
   cccattctcc gccccatggc tgactaattt tttttattta tgcagaggcc gaggccgcct 4260
   ctgcctctga gctattccag aagtagtgag gaggcttttt tggaggccta ggcttttgca 4320
   aaaagctccc gggagcttgt atatccattt tcggatctga tcaagagaca ggatgaggat 4380
   cgtttcgcat gattgaacaa gatggattgc acgcaggttc tccggccgct tgggtggaga 4440
   ggctattcgg ctatgactgg gcacaacaga caatcggctg ctctgatgcc gccgtgttcc 4500
   ggctgtcagc gcaggggcgc ccggttcttt ttgtcaagac cgacctgtcc ggtgccctga 4560
   atgaactgca ggacgaggca gcgcggctat cgtggctggc cacgacgggc gttccttgcg 4620
   cagctgtgct cgacgttgtc actgaagcgg gaagggactg gctgctattg ggcgaagtgc 4680
   cggggcagga tctcctgtca tctcaccttg ctcctgccga gaaagtatcc atcatggctg 4740
   atgcaatgcg gcggctgcat acgcttgatc cggctacctg cccattcgac caccaagcga 4800
   aacatcgcat cgagcgagca cgtactcgga tggaagccgg tcttgtcgat caggatgatc 4860
   tggacgaaga gcatcagggg ctcgcgccag ccgaactgtt cgccaggctc aaggcgcgca 4920
   tgcccgacgg cgaggatctc gtcgtgaccc atggcgatgc ctgcttgccg aatatcatgg 4980
   tggaaaatgg ccgcttttct ggattcatcg actgtggccg gctgggtgtg gcggaccgct 5040
   atcaggacat agcgttggct acccgtgata ttgctgaaga gcttggcggc gaatgggctg 5100
   accgcttcct cgtgctttac ggtatcgccg ctcccgattc gcagcgcatc gccttctatc 5160
   gccttcttga cgagttcttc tgagcgggac tctggggttc gaaatgaccg accaagcgac 5220
   gcccaacctg ccatcacgag atttcgattc caccgccgcc ttctatgaaa ggttgggctt 5280
   cggaatcgtt ttccgggacg ccggctggat gatcctccag cgcggggatc tcatgctgga 5340
   gttcttcgcc caccccaact tgtttattgc agcttataat ggttacaaat aaagcaatag 5400
   catcacaaat ttcacaaata aagcattttt ttcactgcat tctagttgtg gtttgtccaa 5460
   actcatcaat gtatcttatc atgtctgtat accgtcgacc tctagctaga gcttggcgta 5520
   atcatggtca tagctgtttc ctgtgtgaaa ttgttatccg ctcacaattc cacacaacat 5580
   acgagccgga agcataaagt gtaaagcctg gggtgcctaa tgagtgagct aactcacatt 5640
   aattgcgttg cgctcactgc ccgctttcca gtcgggaaac ctgtcgtgcc agctgcatta 5700
   atgaatcggc caacgcgcgg ggagaggcgg tttgcgtatt gggcgctctt ccgcttcctc 5760
   gctcactgac tcgctgcgct cggtcgttcg gctgcggcga gcggtatcag ctcactcaaa 5820
   ggcggtaata cggttatcca cagaatcagg ggataacgca ggaaagaaca tgtgagcaaa 5880
   aggccagcaa aaggccagga accgtaaaaa ggccgcgttg ctggcgtttt tccataggct 5940
   ccgcccccct gacgagcatc acaaaaatcg acgctcaagt cagaggtggc gaaacccgac 6000
   aggactataa agataccagg cgtttccccc tggaagctcc ctcgtgcgct ctcctgttcc 6060
   gaccctgccg cttaccggat acctgtccgc ctttctccct tcgggaagcg tggcgctttc 6120
   tcatagctca cgctgtaggt atctcagttc ggtgtaggtc gttcgctcca agctgggctg 6180
   tgtgcacgaa ccccccgttc agcccgaccg ctgcgcctta tccggtaact atcgtcttga 6240
   gtccaacccg gtaagacacg acttatcgcc actggcagca gccactggta acaggattag 6300
   cagagcgagg tatgtaggcg gtgctacaga gttcttgaag tggtggccta actacggcta 6360
   cactagaaga acagtatttg gtatctgcgc tctgctgaag ccagttacct tcggaaaaag 6420
   agttggtagc tcttgatccg gcaaacaaac caccgctggt agcggttttt ttgtttgcaa 6480
   gcagcagatt acgcgcagaa aaaaaggatc tcaagaagat cctttgatct tttctacggg 6540
   gtctgacgct cagtggaacg aaaactcacg ttaagggatt ttggtcatga gattatcaaa 6600
   aaggatcttc acctagatcc ttttaaatta aaaatgaagt tttaaatcaa tctaaagtat 6660
   atatgagtaa acttggtctg acagttacca atgcttaatc agtgaggcac ctatctcagc 6720
   gatctgtcta tttcgttcat ccatagttgc ctgactcccc gtcgtgtaga taactacgat 6780
   acgggagggc ttaccatctg gccccagtgc tgcaatgata ccgcgagacc cacgctcacc 6840
   ggctccagat ttatcagcaa taaaccagcc agccggaagg gccgagcgca gaagtggtcc 6900
   tgcaacttta tccgcctcca tccagtctat taattgttgc cgggaagcta gagtaagtag 6960
   ttcgccagtt aatagtttgc gcaacgttgt tgccattgct acaggcatcg tggtgtcacg 7020
   ctcgtcgttt ggtatggctt cattcagctc cggttcccaa cgatcaaggc gagttacatg 7080
   atcccccatg ttgtgcaaaa aagcggttag ctccttcggt cctccgatcg ttgtcagaag 7140
   taagttggcc gcagtgttat cactcatggt tatggcagca ctgcataatt ctcttactgt 7200
   catgccatcc gtaagatgct tttctgtgac tggtgagtac tcaaccaagt cattctgaga 7260
   atagtgtatg cggcgaccga gttgctcttg cccggcgtca atacgggata ataccgcgcc 7320
   acatagcaga actttaaaag tgctcatcat tggaaaacgt tcttcggggc gaaaactctc 7380
   aaggatctta ccgctgttga gatccagttc gatgtaaccc actcgtgcac ccaactgatc 7440
   ttcagcatct tttactttca ccagcgtttc tgggtgagca aaaacaggaa ggcaaaatgc 7500
   cgcaaaaaag ggaataaggg cgacacggaa atgttgaata ctcatactct tcctttttca 7560
   atattattga agcatttatc agggttattg tctcatgagc ggatacatat ttgaatgtat 7620
   ttagaaaaat aaacaaatag gggttccgcg cacatttccc cgaaaagtgc cacctgacgt 7680
   c 7681

## Description

### Technical Field

The present invention relates to novel lymphangiogenesis-promoting agents comprising, as active ingredients, HGFs or nucleic acids encoding HGFs, and/or methods for promoting lymphangiogenesis. The present invention also relates to agents or methods for preventing or treating lymphedema. The present invention further relates to methods of screening for compounds having lymphangiogenesis-promoting activity or compounds having preventive or therapeutic effects on lymphedema.

### Background Art

Lymphedema refers to a condition characterized by the occlusion of lymphatic vessels which, in turn, causes abnormal congestion of tissue fluid which results in swelling, chronic inflammation, and/or fibrosis. There are primary and secondary lymphedemas. Known instances of primary lymphedema include Milroy's disease, Meige's disease, distal hypoplasia, proximal obstructive lymphadenopathy, and lymphangiectasia. Secondary lymphedema arises from another disease; for example, it often appears as an aftereffect of surgical treatment of cancers. In particular, secondary lymphedema often occurs after surgery or radiation therapy for breast cancer, uterine cancer, prostatic cancer, Kaposi's sarcoma and such. Particularly, after breast cancer surgery, lymphedema often appears in the upper limbs. 80% or more cases of lymphedema of the upper limbs arise after breast cancer surgery. Furthermore, lymphedema of the upper limbs is presumed to develop at a frequency of several percent or more after breast cancer surgery. In contrast, lymphedema of the lower limbs is often observed subsequent to uterine cancer surgery. In addition to the above, infections, injuries such as burn injury, inflammations and the like can also result in secondary lymphedema.

Lymphedema significantly impairs motor functions and increases the risk of infection in the affected areas, both of which result in reduction of patients' QOL. However, common therapies for lymphedema, such as massage, exercise therapy, and wearing a supporter, merely treat symptoms; neither radical treatment methods nor therapeutic agents are available at present and almost no therapeutic agent is known. While a compound called "guaifenesin" has been reported to be effective in treating lymphedema, its therapeutic effect still remains unclear (Patent Document 1).

VEGF-C, a member of the VEGF family, has been reported to be a peptidic factor that enhances lymphangiogenesis (Patent Documents 2 and 3; Non-Patent Documents 1 and 2). However, the other VEGF family members have no lymphangiogenesis-promoting activity. VEGF-C is the only lymphangiogenesis-promoting factor that is currently known.

Meanwhile, VEGF₁₆₅ (also referred to as "VEGF-A" or more simply "VEGF"; hereinafter also referred to as "VEGF"), is a member of the VEGF family that, like VEGF-C, is also well known as an angiogenic factor; the factor has been also known as a factor that enhances vascular permeability (for example, see Senger, D. et al., Science 219, 983-6 (1983)). In fact, VEGF has been reported to induce edema by its vascular permeability-enhancing activity when overexpressed in tissues (for example, see Isner, JM., et al., Circ. Res. 89: 389-400 (2001)). In fact, edema has been reported to be observed at a frequency of 30% or more in human patients with ischemic diseases of lower limbs when the VEGF gene is intraarterially or intramuscularly introduced into the ischemic areas in lower limbs for the therapeutic purpose (Baumgartner, I. et al., Ann. Intern. Med. 132: 880-884 (2000)). In addition, there are many reports of the increased risk of edema accompanying VEGF gene therapy, including a report showing that edema of the lower limbs was observed in six of nine patients in which a plasmid encoding naked VEGF was administered to ischemic areas of the lower limbs by intramuscular injection (Baumgartner, I. et al., Circulation 97: 1114-1123 (1998)) and another report showing that edema appeared in three limbs when a plasmid encoding naked VEGF was intramuscularly administered to ischemic areas in seven limbs of six patients with lower limb ischemia due to thromboangiitis obliterans (TAO) (Isner, JM., et al., J. Vasc. Surg. 28: 964-975 (1998)).

In addition, VEGF-C is a ligand for VEGF receptor 3 (VEGFR-3), and the signal from VEGFR-3 alone is known to be sufficient for lymphangiogenesis. VEGF-C also binds to VEGFR-2. Since VEGFR-2-deficient mice die earlier than VEGF-A-deficient mice, VEGF-C. and other VEGF members are presumed to be able to complement the VEGF activity (Scavelli, C. et al., J. Anat. 433-449 (2004)). VEGF exerts its function through binding to VEGFR-1 and VEGFR-2. Thus, the activation of VEGFR-2 is thought to induce the enhancement of vascular permeability (for example, see Issbrucker, K. et al., FASEB J. express article 10.1096/fj.02-0329fje. Published online December 18, 2002).

HGF is hepatocyte growth factor, and has not only the activity of promoting the growth of hepatocytes but also other various physiological activities, including angiogenic activity (for example, see Non-Patent Document 3). HGFs are presently applied to the treatment of ischemic disease based on their angiogenic activity (Patent Documents 4 and 5; Non-Patent Document 4).

Kajiya K, et al., Journal of Investigative Dermatology 124 (4), Suppl. S, page A1 (2005) reports that hepatocyte growth factor promotes lymphatic vessel formation and function.

WO2006/105511 discloses methods and compositions for the modulation of hepatocyte growth factor activity to regulate lymphatic vessel development and function.

Yoon Young-Sup, et al., J. Clin. Invest. 111(5) :717-725. (2003) reports that VEGF-C gene therapy augments.postnatal lymphangiogenesis and ameliorates secondary lymphedema.
[Patent Document 1] WO03/000242
[Patent Document 2] US Patent No. 6,818,220
[Patent Document 3] US Patent No. 6,689,352
[Patent Document 4] WO97/07824
[Patent Document 5] WO01/32220
[Non-patent Document 1] Szuba, A. et al., FASEB J. express article 10.1096/fj.02-0401 fje. Published online October 18, 2002
[Non-patent Document 2] Young-sup, Yoo et al., J. Clin. Invest. 111:717-725 (2003)
[Non-patent Document 3] Nakamura Y. et al., J Hypertens. Sep; 14(9):1067-72 (1996)
[Non-patent Document 4] Taniyama Y., et al., Gene Therapy 8, 181-189 (2001)

### Disclosure of the Invention

### [Problems to be Solved by the Invention]

The present invention was achieved in view of the circumstances described above. An objective of the present invention is to provide novel uses of HGFs and genes encoding the same. More specifically, an objective of the present invention is to provide novel therapeutic agents and treatment methods for lymphedema, a condition for which no effective therapeutic agent or treatment method is presently available.

### [Means for Solving the Problems]

The present inventors conducted dedicated studies to achieve the objectives described above. The present inventors searched for factors that could reduce or eliminate lymphedema, and found that HGFs significantly reduced lymphedema. Then, the present inventors discovered that HGFs had lymphangiogenic activity to induce the growth of lymphatic endothelial cells.

As described above, there are many reports associating VEGF gene therapy with an increased risk of inducing edema. Thus, the present inventors considered that VEGF to be unsuitable for lymphedema treatment. Furthermore, since it is suggested that VEGF-C may enhance cell permeability by binding to VEGF receptors other than VEGFR-3, the present inventors considered that the application of VEGF-C to lymphedema treatment also constituted a high risk.

HGF has been known as an angiogenic factor. VEGF is also known as an angiogenic factor, but is presumed to have neither lymphangiogenic activity (see, for example, Dev. Biol. 1997, 188: 96-109) nor the effect of reducing lymphedema, as described above. In view of the above, the novel findings by the present inventors that HGFs have lymphangiogenic activity and the effect of reducing lymphedema is a remarkable discovery.

To confirm the lymphangiogenic activity of HGFs based on its action mechanism, the present inventors demonstrated that the HGF receptor c-met was expressed in lymphatic endothelial cells and that phosphorylation of MAPK and Akt, which are intracellular signaling proteins whose phosphorylation is known to be induced by HGFs, was also induced in lymphatic endothelial cells.

These findings demonstrate that HGFs and their genes are effective as therapeutic or preventive agents for lymphedema, and as lymphangiogenesis-promoting agents. Specifically, present invention provides a vector as defined in the claims, for use in preventing or treating lymphedema; and the use of a vector as defined in the claims for producing a pharmaceutical agent for preventing or treating lymphedema.

### Brief Description of the Drawings

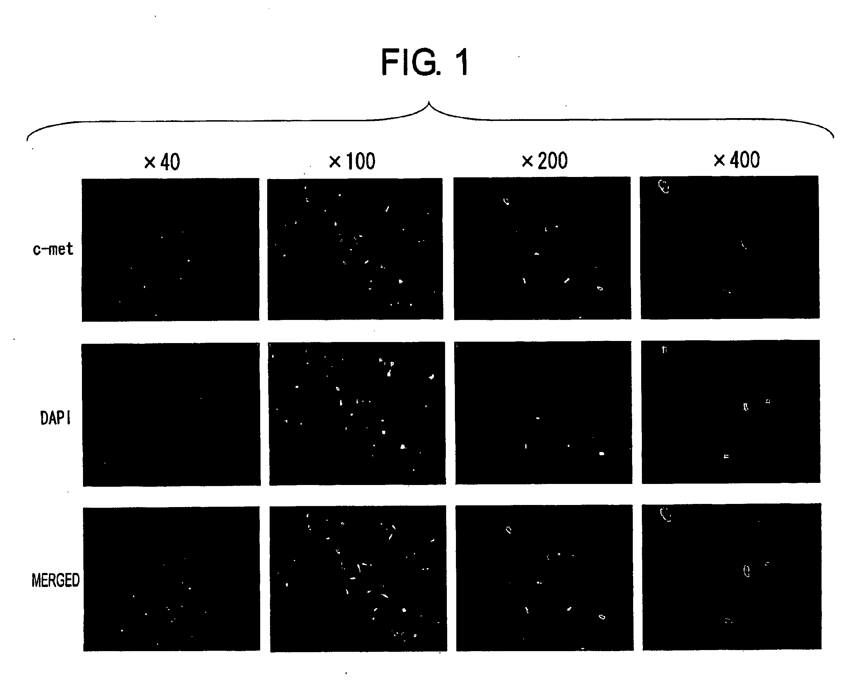

Fig. 1 depicts a series of photographs showing the results of immunofluorescence staining of lymphatic endothelial cells. The top panels correspond to staining with anti-c-met antibody; the middle panels correspond to nuclear staining with DAPI; and the bottom panels constitute merged images of the two. The results confirm that all cells expressed c-met.

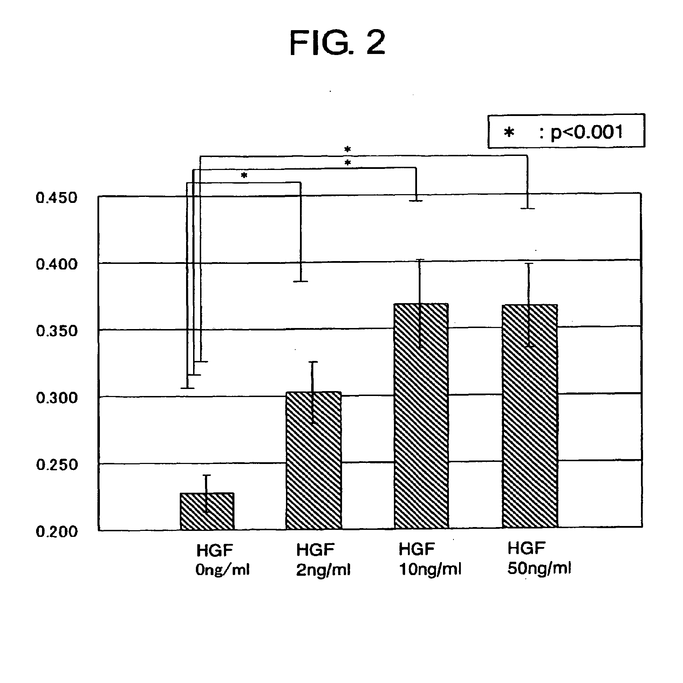

Fig. 2 is a graph depicting the results on an MTS assay examining the effect of a recombinant human HGF on growth capacity. The vertical axis indicates the measured values. The results demonstrate that the HGF promotes the growth of lymphatic endothelial cells in a concentration-dependent manner.

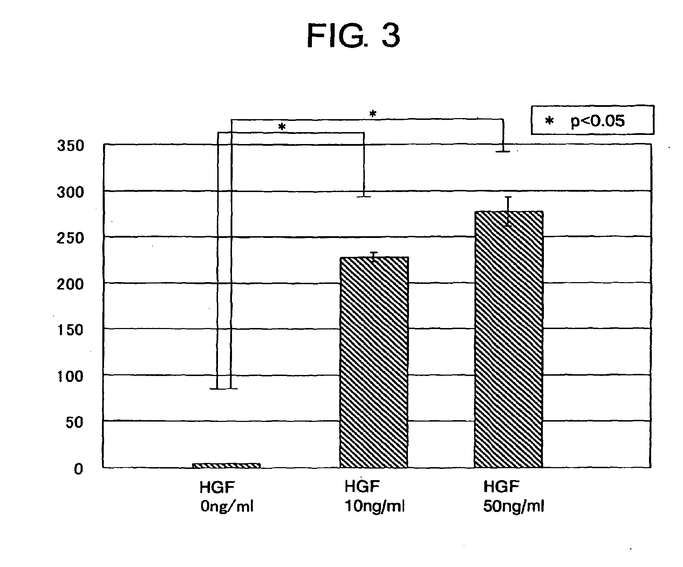

Fig. 3 is a graph depicting the results of a migration assay examining the effect of a recombinant human HGF on migratory capacity. The vertical axis indicates the number of cells. The results demonstrate that the HGF promotes the migration of lymphatic endothelial cells.

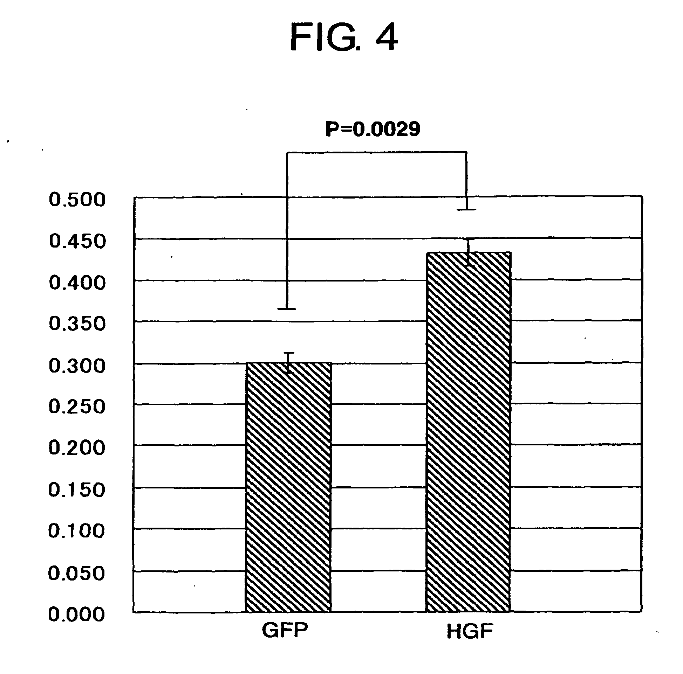

Fig. 4 is a graph depicting the results of an MTS assay examining the effect of introducing a naked cDNA plasmid on growth capacity. The vertical axis indicates the measured values. The results demonstrate that the introduction of the HGF gene is effective to promote the growth of lymphatic endothelial cells.

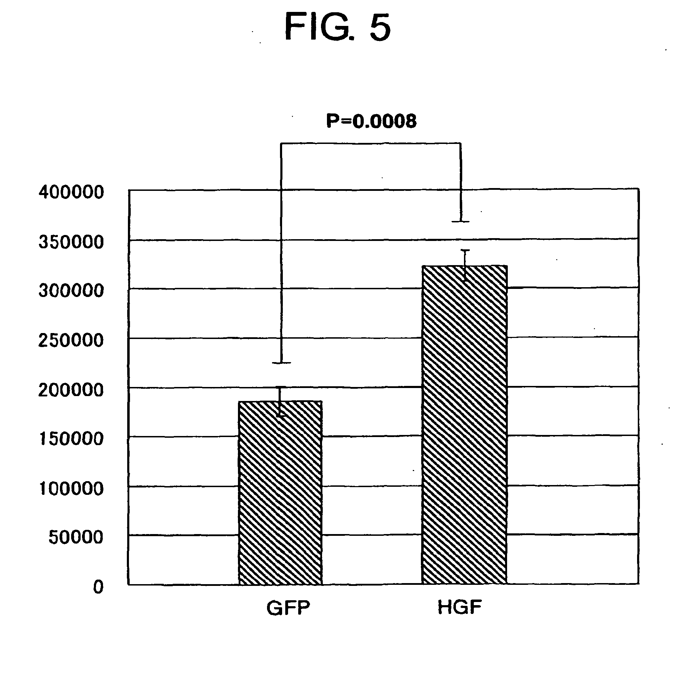

Fig. 5 is a graph depicting the results of a c-fos promoter assay examining the effect of introducing a naked cDNA plasmid on growth capacity. The vertical axis indicates the measured values for luciferase activity. The results demonstrate that the introduction of the HGF gene is effective to promote the growth of lymphatic endothelial cells.

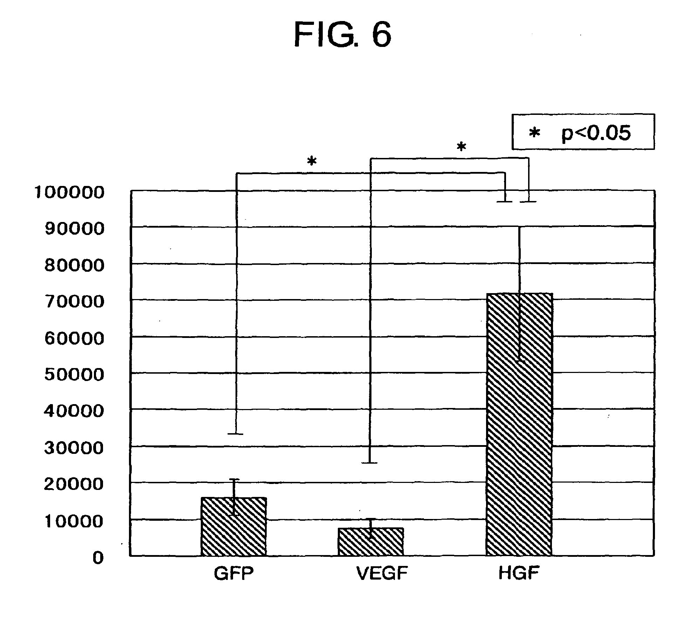

Fig. 6 is a graph depicting the results of a c-fos promoter assay examining the effect of introducing a naked cDNA plasmid on growth capacity. The vertical axis indicates the measured values for luciferase activity. These results demonstrate that the expression vector for HGF cDNA significantly enhances the c-fos promoter activity while the expression vector for VEGF cDNA does not enhance the c-fos promoter activity.

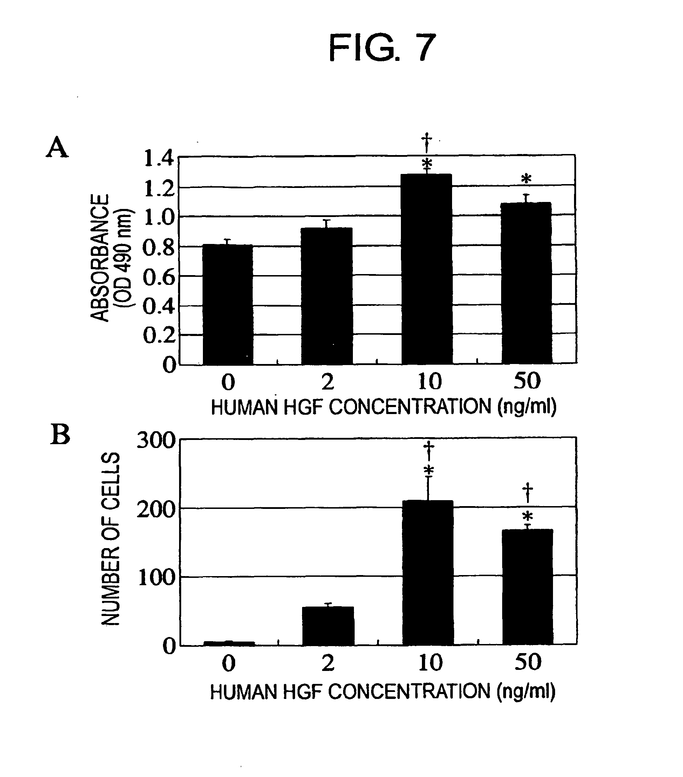

Fig. 7 is a series of graphs demonstrating the effects of HGF on the growth and migratory activity of human lymphatic endothelial cells. Fig. 7A depicts the results of an MTS assay examining cell growth; and Fig. 7B depicts the results of a migration assay examining migratory activity. Each assay was carried out after the recombinant human HGF was added at a concentration of 0, 2, 10, or 50 ng/ml to the culture media of human lymphatic endothelial cells. In Fig. 7A, the vertical axis indicates the absorbance at 490 nm; and in Fig. 7B the vertical axis indicates the number of cells. These results demonstrate that the addition of HGF promotes the growth and migratory activity. *p<0.001 (relative to 0 ng/ml of the recombinant human HGF); †p<0.001 (relative to 2 ng/ml of the recombinant human HGF).

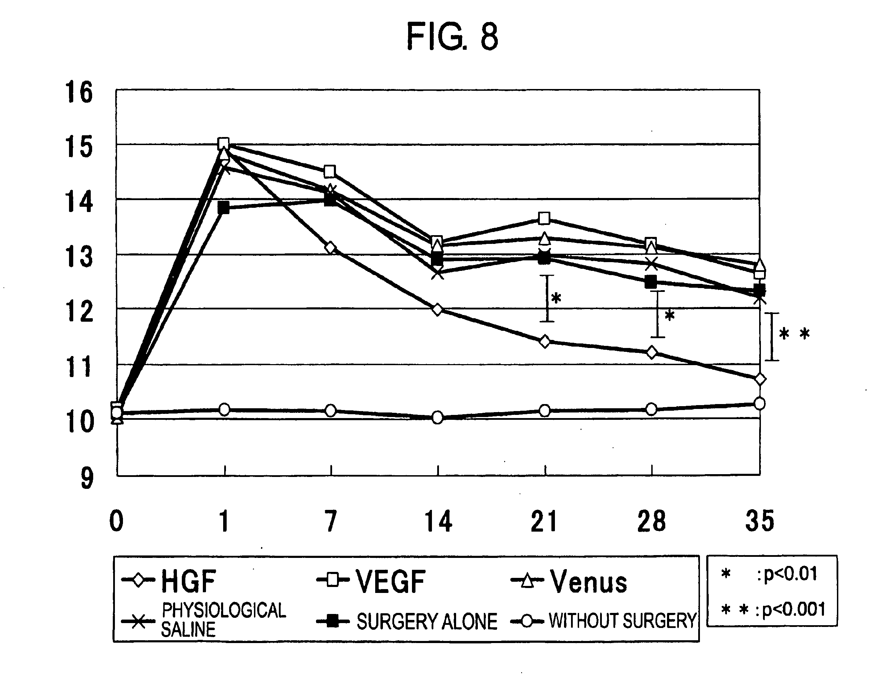

Fig. 8 is a graph depicting the lymphedema-improving effect of the expression plasmid for HGF cDNA in rat models of lymphedema which have lymphedema at the base of their tail. The horizontal axis indicates time elapsed after surgery (days), and the vertical axis indicates the thickness of the base of tail (mm). The Venus group as a control, HGF group, and VEGF group were injected with 0.1 ml of 200 µg expression plasmids for GFP, HGF, and VEGF, respectively. The physiological saline group was injected with 0.1 ml of physiological saline. The results demonstrate that the reduction of tail thickness is promoted by introducing the naked HGF cDNA and thus lymphedema is improved.

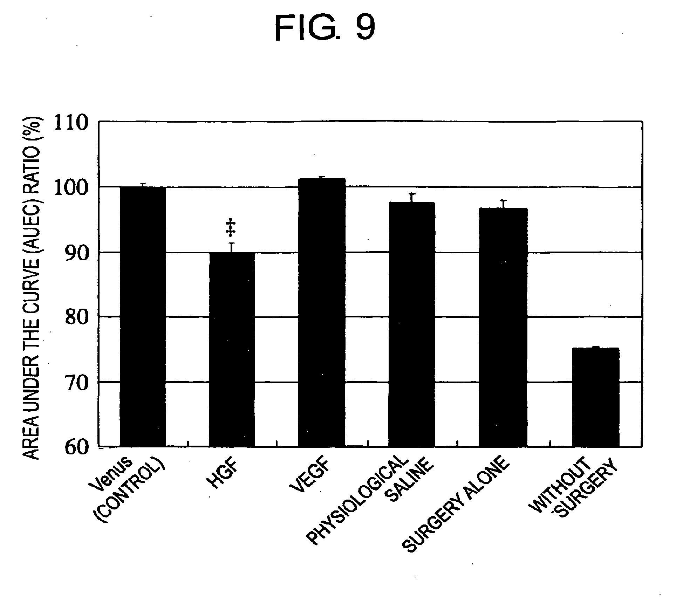

Fig. 9 is a graph obtained by calculating the area under the curve of the graph shown in Fig. 8. The results demonstrate that the tail thickness was significantly reduced only in the group of rats into which the HGF gene was introduced. The vertical axis indicates the area ratio (%) relative to the control. ‡p<0.0001 (relative to each of the group introduced with the VEGF gene, the Venus group, the physiological saline group, and the group treated with surgery alone).

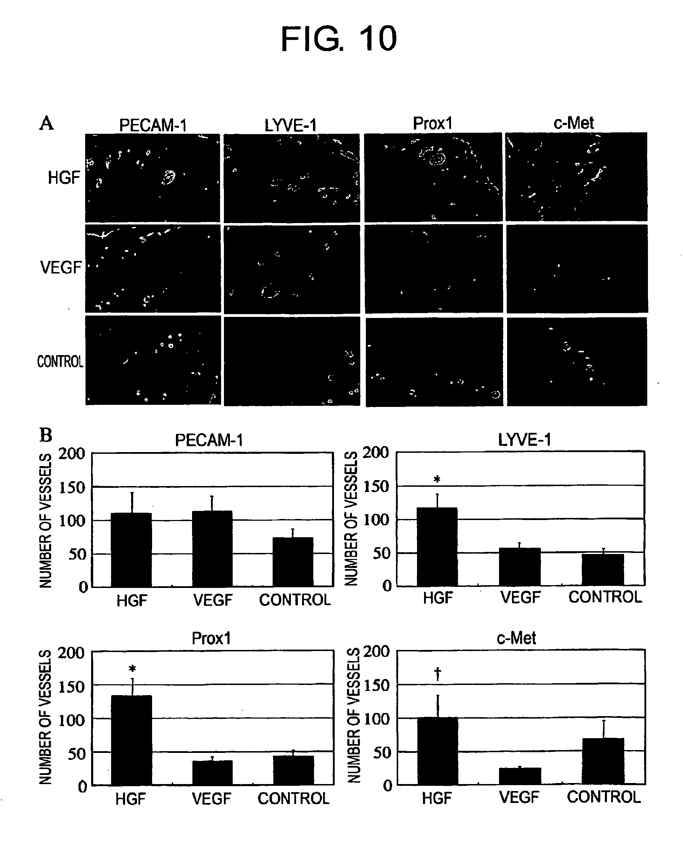

Fig. 10 is a series of photographs and graphs depicting the results of examination on the lymphangiogenesis at the surgical sites after the introduction of the HGF or VEGF gene in rat models of lymphedema. Fig. 10A is composed of micrographs of immunostained sections of the tissue near the gene injection site in each rat. Immunostaining was carried out using antibodies against PECAM-1, LYVE-1, Prox1, and c-Met. The top panels are from rats into which the HGF gene was introduced; the middle panels are from rats into which the VEGF gene was introduced; and the bottom panels are from control rats injected with physiological saline. Fig. 10B is composed of graphs depicting the number of vessels that were positive for immunostaining with each antibody. HGF: rats into which the HGF gene was introduced; VEGF: rats into which the VEGF gene was introduced; control: rats injected with physiological saline. The vertical axis indicates the number of positive vessels. These results demonstrate that the number of vessels positive for the lymphatic endothelial cell markers (LYVE-1 and Proxl) is significantly increased only in the rats introduced with the HGF gene; whereas the number of vessels positive for the endothelial cell marker PECAM-1 is increased in both the rats introduced with the HGF gene and the rats introduced with the VEGF gene as compared to the control, but there is no significant difference. Furthermore, it is shown that the number of vessels positive for c-Met in the rats introduced with the HGF gene is significantly increased as compared with that in the rats introduced with the VEGF gene and tends to be increased as compared to the control.

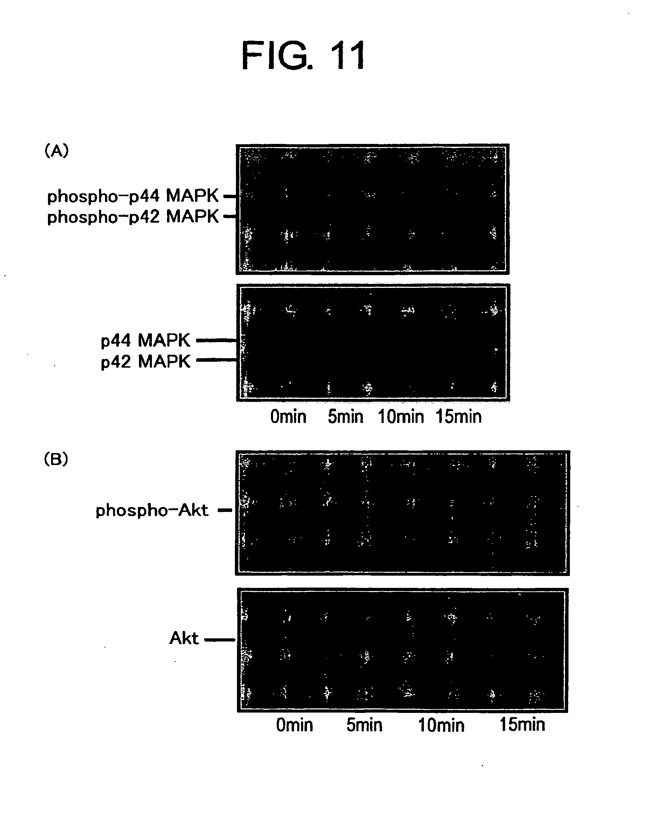

Fig. 11 is a series of photographs depicting phosphorylation of MAPK and Akt after HGF stimulation of canine thoracic duct-derived lymphatic endothelial cells. Figs. 11A and 11B depict results of Western blotting using antibodies specific to phosphorylated MAPK and phosphorylated Akt, respectively (each upper panel). The lower panels show results of Western blotting using antibodies specific to MAPK and Akt regardless of their phosphorylation state. These antibodies were used as internal controls to demonstrate that samples contain almost equal amounts of MAPK or Akt.

### Best Mode for Carrying Out the Invention

The present invention is based on the above-mentioned finding of the present inventors that HGFs activate the growth and migration of lymphatic endothelial cells and thereby promote lymphangiogenesis.

The present invention is further based on the finding that HGFs activate the growth and migration of lymphatic endothelial cells isolated from neither fetal nor neonatal but adult animals and thereby promote lymphangiogenesis. Since lymphatic endothelial cells are differentiated from fetal veins at an early stage of embryogenesis, lymphatic endothelial cell marker proteins are also expressed in venous endothelial cells at the early fetal stage. Lymphatic endothelial cells are also known to further differentiate into venous endothelial cells (Wigle J T and Oliver G. Cell, 98: 769-778 (1999)). Therefore, fetal or neonatal cells are highly likely to be at a stage before terminal differentiation even when expressing lymphatic endothelial cell markers. Thus, fetal or neonatal lymphatic endothelial cells are unlikely to accurately reflect lymphatic endothelial cells of adult animals. The present invention demonstrated the effect of HGFs on lymphatic endothelial cells of adult animals. Thus, HGFs and their genes were found to be effective as therapeutic agents for patients (more particularly adults) suffering from lymphedema after the surgical removal of cancer tissues and/or lymph nodes for cancer treatment and for other lymphedema patients.

As defined in the claims, nucleic acid encoding HGF used in the present invention comprises a nucleic acid encoding an HGF that has been inserted into a plasmid vector, namely pVAX1HGF/MGB1 described in SEQ ID NO: 5.

Nucleic acids of the present invention that encode HGF may be incorporated into vectors, such as cationic liposomes, ligand-DNA complexes, and gene guns. The nucleic acids may be, for example, in a form where they are packaged in HVJ-E vectors derived from Sendai virus envelopes (Kaneda, Y. et al., Mol. Ther. 6, 219-226 (2002)). Alternatively, the nucleic acids may be in a naked form.

In the present invention, the HGF genes may be used alone or in combination with other genes and/or other pharmaceutical agents as needed. In the present invention, the HGF gene and the genes used in combination as needed are incorporated into appropriate vectors that ensure the *in vivo* expression of the genes, and then administered to affected areas of patients.

When lymphangiogenesis-promoting agents comprising as active ingredients nucleic acids encoding HGF are administered, those skilled in the art can readily select an adequate administration route and dose. However, the agents are preferably administered by injection to and/or around the areas where promotion of lymphangiogenesis is desired, in particular by intramuscular injection to the muscles in and/or around the areas. Other administration methods may be used, as long as HGF can be expressed from the administered nucleic acids in and around the areas where lymphangiogenesis is desired to be promoted.

When an active ingredient is a nucleic acid described above, the single dose of the nucleic acid that is effective to promote lymphangiogenesis or to prevent or treat lymphedema can be selected, for example, from the range of 0.001 to 10 mg per kg body weight. Alternatively, when administered to human patients, the dose of the nucleic acid can be selected, for example, from the range of 0.001 to 50 mg/body, and the single dose of the nucleic acid is preferably about 0.5 to 50 mg/body. However, the dose is not limited to those described above.

In a preferred embodiment pVAX1HGF/MGB1 described in SEQ ID NO: 5 dissolved in a physiological buffer or pharmaceutically acceptable carrier is intramuscularly injected to the muscle in and/or around an area where lymphangiogenesis is desired to be promoted in a human patient at a single dose of 1 mg to 20 mg, preferably 1 mg to 10 mg, and more preferably 2 mg to 10 mg (for example, 3 mg to 6 mg, or 2 mg to 4 mg). The agent may be administered twice or more, preferably three times or more, at intervals of about 1 to 10 weeks, preferably about 1 to 8 weeks (for example, 2 to 6 weeks, or 1 to 4 weeks). When administered to humans, the agents may be administered, for example, according to the method described by Morishita R. et al., Hypertension 44(2):203-9 (2004), or an appropriately modified method thereof.

The lymphangiogenesis-promoting agents of the present invention can be used as pharmaceutical agents for preventing or treating lymphedema. Lymphedema refers to a condition where occlusion of lymphatic vessels causes abnormal congestion of tissue fluid, resulting in swelling, chronic inflammation, and/or fibrosis. Lymphedema that is a target for prevention or treatment by the lymphangiogenesis-promoting agents of the present invention include all conditions that have such symptoms as described above, regardless of their names. The lymphangiogenesis-promoting agents of the present invention are useful for preventing or treating diseases involving such symptoms.

In subjects at high risk for lymphedema (for example, patients whose lymph nodes have been extirpated along with malignant tumors by surgery), lymphedema can be prevented by administering the lymphangiogenesis-promoting agents of the present invention.

In accordance with the claims, the invention provides means for preventing or treating lymphedema. The administration site may be any site where lymphangiogenesis is desired to be promoted. Lymphedema can also be treated, for example, by applying the above-described methods to patients with lymphedema. More specifically, lymphangiogenesis can be promoted by administering the lymphangiogenesis-promoting agents, as described above.

Those skilled in the art can administer the human HGF genes to affected areas of patients with lymphedema, appropriately considering the purpose. The administration to the affected areas of patients can be achieved by methods known to those skilled in the art.

In the present invention, the vector described above may be administered alone or in combination with genes encoding other lymphangiogenic factors, or as compositions in combination with pharmaceutically acceptable carriers and/or additives. When administered in a form of composition, specific embodiments of genes encoding other lymphangiogenic factors, and pharmaceutically acceptable carriers and/or additives to be used in combination are as described above.

Affected areas to which the vector described above may be administered are not particularly limited, so long as they are areas in which the symptoms of lymphedema have appeared or are predicted to appear. For example, local administration to the upper arm region can be considered for lymphedema of upper limb, which is often observed after breast cancer surgery and such; alternatively, local administration to the femoral region can be considered for lymphedema of lower limb, which is often observed after uterine cancer surgery and such.

The dose is as described above; however, it varies depending on the type of disease, patient's weight, age, sex, and symptoms, administration purpose, form of the gene to be introduced and such. Those skilled in the art can appropriately determine the dose.

The subjects to which the vector of the present invention, is administered include any mammal, such as a monkey, dog, and cat, in addition to human.

### Examples

Herein below, the present invention will be specifically described with reference to Examples, but is not to be construed as being limited thereto.

### [Example 1] Effect of HGFs on primary cultured lymphatic endothelial cells derived from canine thoracic duct

### (1) Preparation of primary cultured lymphatic endothelial cells derived from the canine thoracic duct

Primary cultured lymphatic endothelial cells derived from the canine thoracic duct were prepared by a known method (Microcirculation 6, 75-78 (1999)). Adult mongrel dogs (including both male and female; 6 to 12 kg) were sacrificed by bleeding from the femoral artery under anesthesia. Then, the thoracic ducts (10 to 15 cm) were isolated and placed in cold (4°C) Hanks' balanced salt solution (HBSS). Connective tissues and adipocytes were removed. Branches of the thoracic ducts were ligated with sterilized silk thread, and the lymphatic vessels were washed with cold HBSS. Next, the thoracic ducts were incubated in a collagenase solution (250 U/ml HBSS) at 37°C for 10 minutes. The ducts were washed with MEM containing 10% fetal bovine serum (FBS) and the cells were collected. After collagenase was removed by centrifugation, the cells were suspended in complete MEM supplemented with 20% FBS and an antibiotic, and then cultured in 35-mm dishes coated with type I collagen. The cells were cultured under a wet condition with 5% CO₂ at 37°C. When the cells reached confluence, they were detached with trypsin/EDTA and collected. Lymphatic endothelial cells were subcloned to prepare lymphatic endothelial cell clones. The clonal cells were able to be passaged at least 10 or more times.

The lymphatic endothelial cells thus obtained were confirmed to be immunostained with different types of lymphatic endothelial cell-specific antibodies (including anti-VEGFR-3 antibody and anti-Proxl antibody). Furthermore, the expression of c-met, an HGF receptor, in these cells was confirmed by immunostaining. This novel finding is attributable to the present invention (Fig. 1).

### (2) Enhancement of growth capacity by a recombinant human HGF

A recombinant human HGF was added at a concentration of 0, 2, 10, or 50 ng/ml to medium containing the lymphatic endothelial cells derived from the canine thoracic duct (70% or more confluent in 96-well plates) prepared above in (1). After three days, the MTS assay was carried out using the CellTiter 96 One Solution Reagent (Promega). The assay was independently performed on nine wells for each concentration. The results are shown in Table 1 and Fig. 2.

**Table 1**

| | HGF 0ng/ml | HGF 2ng/ml | HGF 10ng/ml | HGF 50ng/ml |
|---|---|---|---|---|
| No1 | 0.22 | 0.257 | 0.339 | 0.285 |
| No2 | 0.203 | 0.26 | 0.291 | 0.299 |
| No3 | 0.203 | 0.279 | 0.344 | 0.341 |
| No4 | 0.183 | 0.248 | 0.251 | 0.25 |
| No5 | 0.208 | 0.262 | 0.3 | 0.301 |
| No6 | 0.193 | 0.247 | 0.338 | 0.406 |
| No7 | 0.26 | 0.367 | 0.403 | 0.477 |
| No8 | 0.288 | 0.417 | 0.499 | 0.503 |
| No9 | 0.289 | 0.387 | 0.549 | 0.437 |
| Mean | 0.227 | 0.303 | 0.368 | 0.367 |
| Standard deviation (SD) | 0.041 | 0.068 | 0.099 | 0.092 |
| Standard error (SE) | 0.014 | 0.023 | 0.033 | 0.031 |

Even in the presence of 2 ng/ml of HGF, the measured values significantly increased. These results suggest that the HGF strongly promotes the growth of lymphatic endothelial cells.

### (3) Enhancement of migratory activity by a recombinant human HGF

### (i) Enhancement of migratory activity by a recombinant HGF

The migratory activity of cells was measured by a previously reported method using the Boyden chamber (Arterioscler Thromb Vasc Biol. 22:108-114 (2002)). A polyvinylpyrrolidone (PVP)-free polycarbonate membrane with a pore size of 8 µm (Neuro Probe Inc., Gaithersburg, MD) was coated with 0.1% gelatin and excess gelatin was removed using phosphate buffered saline (PBS). The above-described membrane was placed onto the lower chamber of the Boyden chamber containing 28 µl of EBM2 medium supplemented with 1% FBS, and 50 µl of medium containing 10⁶ of the lymphatic endothelial cells derived from the canine thoracic duct prepared above in (1) was added to the upper chamber. A recombinant human HGF was added to the medium at a concentration of 0, 10, or 50 ng/ml. The cells were cultured under a wet condition with 5% CO₂ at 37°C for four hours. The membrane was removed, and the cells on the upper surface of the membrane were detached. The cells on the lower surface of the membrane were stained with the Diff-Quick (Sysmex, Hyogo, Japan) and counted. This assay was independently performed in three wells for each HGF concentration. The results are shown in Table 2 and Fig. 3

**Table 2**

| | HGF (-) | HGF 10ng/ml | HGF 50ng/ml |
|---|---|---|---|
| No1 | 3 | 224 | 271 |
| No2 | 4 | 238 | 307 |
| No3 | 6 | 222 | 254 |
| Mean | 4.33 | 228.00 | 277.33 |
| SD | 1.53 | 8.72 | 27.06 |
| SE | 0.0882 | 5.033 | 15.624 |

Table 2 shown above and Fig. 3 suggest that even in the presence of 10 ng/ml of HGF, the measured values significantly increases and thus the HGF promotes migration of lymphatic endothelial cells.

The above results suggest that HGFs promote lymphangiogenesis by promoting the growth and migration of lymphatic endothelial cells.

### (ii) Enhancement of migratory activity by a paracrine recombinant HGF

An expression plasmid for human HGF cDNA, pVAX1HGF/MGB1 (SEQ ID NO: 5), was packaged in the HVJ-E vector by a previously reported method (Kaneda, Y. et al., Mol. Ther. 6, 219-226 (2002)).

BHK cells were cultured until they reached confluence in 100-mm dishes. 50 HAU of the vector containing the plasmid was added to the culture medium of the BHK cells and then introduced into the cells. After 24 hours of culture, the cells were transferred into inserts for 24-well plates and cultured until they were almost confluent.

Meanwhile, the lymphatic endothelial cells derived from the canine thoracic duct descried above in (1) were cultured until 70% or more confluent in 24-well plates, and the inserts containing the above-described BHK cells introduced with the expression plasmid for human HGF cDNA pVAX1HGF/MGB1 were placed in the wells. After 48 hours of culture, in the same way as described above, the cells were subjected to the MTS assay. The assay was independently performed on six wells for each sample. The cells treated in the same way using a GFP expression plasmid instead of the expression plasmid for human HGF cDNA pVAX1 HGF/MGB1, were used as a negative control. The results are shown in Table 3 and Fig. 4.

**Table 3**

| | GFP | HGF |
|---|---|---|
| No1 | 0.260 | 0.497 |
| No2 | 0.320 | 0.451 |
| No3 | 0.338 | 0.397 |
| No4 | 0.272 | 0.414 |
| No5 | 0.306 | 0.440 |
| No6 | 0.307 | 0.400 |
| Mean | 0.301 | 0.433 |
| SD | 0.029 | 0.038 |
| SE | 0.012 | 0.016 |

When the cells were co-cultured with the cells introduced with the HGF cDNA, the measured values in the MTS assay were significantly increased as compared to the negative control. These results demonstrate that the introduction of the HGF gene is effective to promote the growth of lymphatic endothelial cells.

To further confirm the promotion of lymphatic endothelial cell growth by the HGF, c-fos promoter activity was measured by a previously reported method (Hypertension 37(2); 581-586 (2001)).

The assay was independently performed on six wells for each sample. The cells treated using a GFP expression plasmid in the same way as described above, were used as a negative control. The results are shown in Table 4 and Fig. 5.

**Table 4**

| | GFP | HGF |
|---|---|---|
| No1 | 231101 | 317234 |
| No2 | 210645 | 344423 |
| No3 | 143101 | 303133 |
| No4 | 141224 | 319125 |
| No5 | 195948 | 384934 |
| No6 | 189940 | 270410 |
| Mean | 185326.50 | 323209.83 |
| SD | 36327.55 | 38738.02 |
| SE | 14830.66 | 15814.73 |

The c-fos promoter activity is known to be positively correlated with the cell growth capacity. Thus, given the fact that the HGF increases the c-fos promoter activity, it is apparent that the introduction of the HGF gene is effective to promote the lymphatic endothelial cell growth.

### (5) Comparison of growth promoting effects between a human HGF cDNA and VEGF cDNA in an autocrine system

Using Lipofectamine Plus (GIBCO-BRL), an expression plasmid for a naked human HGF cDNA, pVAX1HGF/MGB1, and a c-fos luciferase reporter gene plasmid (p2FTL) for c-fos promoter assay were introduced into the above-described lymphatic endothelial cells cultured until subconfluent in 6-well plates (J. Hypertens. 16: 993-1000 (1998)). After 24 hours of culture, the cells were cultured in serum-free culture medium for 24 hours. The luciferase activity was then determined by a conventional method, and found to be increased. Next, using the human VEGF cDNA instead of the human HGF cDNA, the same experiments as described above in (4) were carried out to compare the effects of human HGF cDNA and VEGF cDNA on the c-fos promoter activity. The assay was independently performed on four wells for each sample. The results are shown in Table 5 and Fig. 6.

**Table 5**

| | GFP | VEGF | HGF |
|---|---|---|---|
| No1 | 28135 | 9213 | 120035 |
| No2 | 19431 | 14273 | 80231 |
| No3 | 6789 | 3728 | 46335 |
| No4 | 9085 | 2936 | 39688 |
| Mean | 15860.00 | 7537.50 | 71572.25 |
| SD | 9859.20 | 5287.10 | 36865.23 |
| SE | 4929.599 | 2643.551 | 18432.617 |

These results suggest that introduction of the expression plasmid for HGF cDNA significantly increases the c-fos promoter activity through the autocrine of HGF, while the expression plasmid for VEGF cDNA does not increase the c-fos promoter activity and thus does not promote the lymphatic endothelial cell growth.

The results of experiments using the primary cell culture system described above suggest that lymphangiogenesis is promoted by HGFs, that lymphangiogenesis can be promoted by introducing the HGF gene, and that the introduction of an expression plasmid for a naked HGF cDNA, namely the introduction of a nucleic acid encoding an HGF, is effective for the promotion.

Furthermore, lymphatic endothelial cells isolated from adult dogs were used in the present Example, and thus HGFs were demonstrated to have the above-described activity on lymphatic endothelial cells of adult animals. This suggests that HGFs are useful as therapeutic agents for lymphedema.

Moreover, in order to confirm that the lymphangiogenesis-promoting activity of human HGF was not specific to canine lymphatic endothelial cells, the growth-promoting activity of HGF on primary cultured aortic and venous endothelial cells isolated from adult mongrel dogs in the same way as described above was investigated. As a result, the human HGF was found to have growth-promoting activity on these cells. Furthermore, this growth-promoting activity was comparable to that on equivalent human cells (data not shown). Consequently, human HGFs also act on canine vascular endothelial cells in the same manner as on human vascular endothelial cells. These findings suggest that the lymphangiogenesis-promoting activity of human HGFs is not specific to canine lymphatic endothelial cells.

### (6) Confirmation of HGF activity on human lymphatic endothelial cells

It was postulated that human lymphatic endothelial cells would give the same result as that obtained using the lymphatic endothelial cells isolated form adult dogs. To confirm this prediction, the effect of HGF on the growth and migratory capacities of human lymphatic endothelial cells was assayed (AngioBio Co. (Del Mar, CA)).

The cells at passage 5 to 8 were used in the experiments. By immunostaining, it was confirmed that von Willebrand factor, VEGF receptor-3, Prox1, and c-Met, which are lymphatic endothelial cell markers, were also expressed in the cells (data not shown). The MTS assay and migration assay were carried out in the same way as the experiments using canine lymphatic endothelial cells.

The results of MTS assay and migration assay are shown in Figs. 7A and 7B, respectively. Both cell growth capacity and migratory capacity were increased by adding a recombinant human HGF. They were significantly increased at 10 ng/ml or higher concentrations as compared to the cells in the absence of HGF.

The results confirm the initial prediction that an HGF would promote lymphangiogenesis of human lymphatic endothelial cells as well as the lymphatic endothelial cells from adult dogs described above in (1) to (5).

The above results suggest that HGFs have lymphangiogenesis-promoting activity not only on dogs but also on other mammals (including humans).

### [Example 2] Effect of HGFs on rat models of lymphedema

Based on the findings described above, the effect of an HGF on lymphedema was tested using rat models to demonstrate the *in vivo* effect.

### (1) Preparation of rat models of lymphedema

Rat models of Lymphedema were prepared according to Slavin SA et al. (Anals of Surgery 229, 421-427 (1999)). To confirm the presence of lymphedema in the tail of rat models, physiological saline containing 0.5% Patent Blue dye was injected at a position several centimeters distant from the base of tail immediately before surgery. The tail region was dissected on the day following the surgery, and the blue dye was observed in the lymphatic vessels (data not shown). Furthermore, the base of tail was evidently thicker as compared to control rats, and thus the above rats were demonstrated to be useful as lymphedema models.

### (2) Lymphedema-improving effect of an expression plasmid for HGF cDNA on rat models of lymphedema

The effect of introduction of the human HGF gene on lymphedema was investigated using the rat models prepared as described above.

200 µg (/100 µl) of an expression plasmid for a naked human HGF cDNA, pVAX1HGF/MGB1, and 200 µg (/100 µl) of an expression plasmid for a naked VEGF cDNA, and as a control, 200 µg (/100 µl) of a naked GFP expression plasmid (Venus plasmid) were intramuscularly administered one, seven, and 14 days after surgery. In the group with surgery alone, only surgery was carried out without injection. In the group without surgery, no surgery was carried out. In the physiological saline group, 100 µl of physiological saline was intramuscularly injected one, seven, and 14 days after surgery. The tail thickness in each group was measured every seven days up to day 35 after surgery. Five rats in each group were tested and the mean was determined, which is shown in Fig. 8.

In all groups, the tail thickness transiently increased after surgery. However, the tail thickness was more rapidly decreased and the degree was greater only in the group introduced with HGF cDNA as compared to the other groups. This difference was significant after day 21. Thus, lymphedema was found to be improved by introducing the naked HGF cDNA plasmid.

The areas under the curves shown in Fig. 8 were determined. The result is shown in Fig. 9. There was no difference between rats introduced with the VEGF gene and rats in the control Venus group; however, the tail thickness was found to be significantly decreased in rats introduced with the HGF gene. This decrease in the thickness of tail affected with lymphedema implies the relief or cure of lymphedema. Thus, lymphedema was clearly demonstrated to be relieved or cured by introducing a nucleic acid encoding an HGF (the HGF gene).

Tissue samples were collected from the above-described surgical site in the rat tails on days 4, 10, and 17 after surgery, and human HGF mRNA level was determined by real-time RT-PCR using a conventional method. As a result, human HGF expression was confirmed up to day 17 after surgery only in the group introduced with the human HGF gene (data not shown).

Furthermore, in the rats introduced with the HGF gene, rats introduced with the VEGF gene, and control rats injected with physiological saline, expression of endothelial cell marker (PECAM-1), lymphatic endothelial cell markers (LYVE-1 and Proxl), and c-met was detected by immunostaining at the injection sites on day 35 after surgery. Typical staining images are shown in Fig. 10 (Fig. 10A). In addition, the expression level of each marker was determined by counting the number of immunostaining-positive vessels in microscopic fields randomly selected using a known method (Yoon YS, et al., J. Clin. Invest. 111: 717-725 (2003)) (Fig. 10B). This result showed that there was no difference in the number of vessels positive for an endothelial cell marker, PECAM-1, in both rats introduced with the HGF gene and rats introduced with the VEGF gene as compared to the control. On the other hand, the numbers of vessels positive for lymphatic endothelial cell markers, LYVE-1 and Prox1, were both significantly increased in the rats introduced with the HGF gene, while there was no difference between the control and rats introduced with the VEGF gene. Furthermore, the number of vessels positive for c-met also tended to increase only in the rats introduced with the HGF gene as compared to the control. These results confirm that the HGF gene promotes lymphangiogenesis but the VEGF gene does not.

The results obtained with the rat models of lymphedema demonstrate that when a nucleic acid encoding an HGF is injected and expressed near sites affected with lymphedema, lymphatic vessels are newly generated around the injection sites. The rat models can reflect any type of lymphedema. Thus, the findings obtained herein with the rat models, that the symptom of lymphedema is relieved or cured *in vivo* by administering the HGF gene, suggest that HGFs and their genes are useful as therapeutic agents for lymphedema in mammals including humans.

### [Example 3] Confirmation of HGF signaling in lymphatic endothelial cells

To confirm the above-described mechanism of action of HGFs, it was demonstrated that phosphorylation of MAPK and Akt, which is known to be induced by HGFs in vascular endothelial cells, was also induced in lymphatic endothelial cells by HGF stimulation. The phosphorylation of MAPK and Akt is known to be essential for vascular endothelial cell growth promoted by HGFs (Nakagami H., Hypertension 37[part 2]: 581-586 (2001)).

The culture medium of the above-described lymphatic endothelial cells derived from canine thoracic duct was changed with MEM containing 0.5% FCS or FCS-free MEM 12 hours or more before addition of an HGF. A recombinant human HGF was added at a concentration of 100 ng/ml, and after 0 to 15 minutes the medium was removed and the cells were lysed with lysis buffer (50 mM Tris-Cl, 2.5 mM EGTA, 1 mM EDTA, 10 nM NaF, 1% deoxycorticosterone, 1% Triton X-100, 1 nM PMSF, and 2 mM sodium vanadate (pH 7.5)). The genome molecules were disrupted by sonication. Samples containing 20 µg proteins were subjected to 10% SDS-PAGE according to a conventional method. After transfer to nitrocellulose membrane, Western blotting was carried out using an anti-MAPK/ERK antibody, antibody specific to phosphorylated MAPK/ERK (phosphospecific; Tyr705 or Ser727), anti-Akt antibody, and antibody specific to phosphorylated Akt. The used primary antibodies were available from Cell Signaling Technology and others. Detection was achieved using the ECL kit (Amersham).

The results are shown in Fig. 11. Fig. 11A shows a result of Western blotting for MAPK. The result demonstrate that the phosphorylation of both p44 and p42 MAPKs is enhanced within five minutes after HGF stimulation. The result of Akt is shown in Fig. 11B. Likewise, the phosphorylation of Akt is enhanced within five minutes after HGF stimulation. Each bottom panel depicts detection of p44 and p42 MAPKs, or Akt as an internal control.

The phosphorylation of MAPK and Akt was also enhanced in lymphatic endothelial cells in response to HGF stimulation, as described above. This suggests that, in lymphatic endothelial cells, HGFs also induce, via c-met, the same phosphorylation cascade as in vascular endothelial cells. Thus, the phosphorylation of MAPK and Akt is presumed to be an essential signal for lymphatic endothelial cell growth.

Furthermore, the lymphatic endothelial cell growth-promoting activity of HGFs was found to be reduced by the MEK inhibitors U0126 (50 µM) and PD9805 (30 µM) and the PI3 kinase inhibitors Ly294002 (50 µM) and wortmannin (100 nM) (data not shown). This finding also suggests that the lymphangiogenic activity of HGFs is based on induction via c-met of the same signal cascade as in vascular endothelial cells by HGFs.

### Industrial Applicability

The present invention provides novel lymphangiogenesis-promoting agents. The lymphangiogenesis-promoting agents provided by the present invention comprise human HGFs as active ingredients.

VEGF-C is a known peptidic factor that promotes lymphangiogenesis. However, other VEGF members belonging to the VEGF family have no lymphangiogenesis-promoting activity. Only VEGF-C has been known to have lymphangiogenesis-promoting activity.

Meanwhile, HGFs, which were discovered herein to have lymphangiogenesis-promoting activity, are known as angiogenic factors, like VEGF. Since VEGF has no lymphangiogenesis-promoting activity, those skilled in the art have presumed that HGFs also have no lymphangiogenesis-promoting activity. Accordingly, the findings of the present invention, that HGFs have lymphangiogenic activity, is considered as a remarkable fact.

In addition, VEGF-C may induce edema via cross-linking with VEGFR2; however, HGFs do not have such a risk. This is also a remarkable feature of the lymphangiogenesis-promoting agents of the present invention. The lymphangiogenesis-promoting agents are effective for preventing or treating lymphedema.

Furthermore, HGFs activate the growth and migration of lymphatic endothelial cells isolated not from fetal or neonatal systems but from adult animals, and thereby promote lymphangiogenesis. Most of patients suffering from lymphedema after surgical removal of cancer tissues and/or lymph nodes for cancer treatment are adult. Thus, the agents of the present invention are particularly useful as therapeutic agents for adult suffering from lymphedema after surgical removal of cancer tissues and/or lymph nodes for cancer treatment.

## Claims

1. A vector for use in preventing or treating lymphedema, which is a mammalian expression vector into which a nucleic acid encoding an HGF has been inserted, wherein the vector comprises the nucleotide sequence of SEQ ID NO: 5.

2. Use of a mammalian expression vector into which a nucleic acid encoding an HGF has been inserted in the manufacture of a medicament for preventing or treating lymphedema, wherein the vector comprises the nucleotide sequence of SEQ ID NO: 5.

3. The vector for use of claim 1, wherein the vector is for administration by intramuscular injection to or around an affected area in a subject.

4. The use of claim 2, wherein the vector is for administration by intramuscular injection to or around an affected area in a subject.

5. The vector for use of claim 1, wherein the lymphedema is secondary lymphedema.

6. The vector for use of claim 5, wherein the lymphedema is after surgical removal of cancer tissues and/or lymph nodes for cancer treatment.

7. The use of claim 2, wherein the lymphedema is secondary lymphedema.

8. The use of claim 7, wherein the lymphedema is after surgical removal of cancer tissues and/or lymph nodes for cancer treatment.

## Patentansprüche

1. Vektor zur Verwendung bei der Prävention oder Behandlung von Lymphödem, der ein Säugetierexpressionsvektor ist, in den eine für einen HGF kodierende Nucleinsäure insertiert wurde, worin der Vektor die Nucleotidsequenz aus Seq.-ID Nr. 5 umfasst.

2. Verwendung eines Säugetierexpressionsvektors, in den eine für einen HGF kodierende Nucleinsäure insertiert wurde, zur Herstellung eines Medikaments zur Prävention oder Behandlung von Lymphödem, worin der Vektor die Nucleotidsequenz aus Seq.-ID Nr. 5 umfasst.

3. Vektor zur Verwendung nach Anspruch 1, worin der Vektor der Verabreichung durch intramuskuläre Injektion in oder um eine betroffene Stelle in einem Individuum dient.

4. Verwendung nach Anspruch 2, worin der Vektor der Verabreichung durch intramuskuläre Injektion in oder um eine betroffene Stelle in einem Individuum dient.

5. Vektor zur Verwendung nach Anspruch 1, worin das Lymphödem ein sekundäres Lymphödem ist.

6. Vektor zur Verwendung nach Anspruch 5, worin das Lymphödem nach chirurgischer Entfernung von Krebsgewebe und/oder Lymphknoten zur Krebsbehandlung vorliegt.

7. Verwendung nach Anspruch 2, worin das Lymphödem ein sekundäres Lymphödem ist.

8. Verwendung nach Anspruch 7, worin das Lymphödem nach chirurgischer Entfernung von Krebsgewebe und/oder Lymphknoten zur Krebsbehandlung vorliegt.

## Revendications

1. Vecteur pour utilisation dans la prévention ou le traitement du lymphoedème, qui est un vecteur d'expression de mammifère dans lequel un acide nucléique codant pour un HGF a été introduit, lequel vecteur comprend la séquence nucléotidique de SEQ ID NO: 5.

2. Utilisation d'un vecteur d'expression de mammifère dans lequel un acide nucléique codant pour un HGF a été introduit, dans la fabrication d'un médicament destiné à la prévention ou au traitement du lymphoedème, dans laquelle le vecteur comprend la séquence nucléotidique de SEQ ID NO: 5.

3. Vecteur pour utilisation selon la revendication 1, lequel vecteur est destiné à être administré par injection intramusculaire à ou près d'une zone touchée chez un sujet.

4. Utilisation selon la revendication 2, dans laquelle le vecteur est destiné à être administré par injection intramusculaire à ou près d'une zone touchée chez un sujet.

5. Vecteur pour utilisation selon la revendication 1, où le lymphoedème est un lymphoedème secondaire.

6. Vecteur pour utilisation selon la revendication 5, où le lymphoedème s'est formé après l'exérèse chirurgicale de ganglions lymphatiques et/ou tissus cancéreux pour un traitement anticancéreux.

7. Utilisation selon la revendication 2, dans laquelle le lymphoedème est un lymphoedème secondaire.

8. Utilisation selon la revendication 7, dans laquelle le lymphoedème s'est formé après l'exérèse chirurgicale de ganglions lymphatiques et/ou tissus cancéreux pour un traitement anticancéreux.
